(19) Europäisches Patentamt
European Patent Office
Office européen des brevets

(11) EP 1 296 974 B1

(12) # EUROPEAN PATENT SPECIFICATION

(45) Date of publication and mention of the grant of the patent:
**05.08.2009 Bulletin 2009/32**

(21) Application number: **01984014.9**

(22) Date of filing: **11.06.2001**

(51) Int Cl.:
***C07D 401/12*** *(2006.01)* ***C07D 207/16*** *(2006.01)*
***C07D 403/12*** *(2006.01)* ***C07D 417/12*** *(2006.01)*
***C07D 409/12*** *(2006.01)* ***A61K 31/40*** *(2006.01)*
***A61K 31/4439*** *(2006.01)* ***A61K 31/506*** *(2006.01)*
***A61K 31/428*** *(2006.01)* ***A61K 31/427*** *(2006.01)*
***A61P 3/10*** *(2006.01)*

(86) International application number:
**PCT/EP2001/006595**

(87) International publication number:
**WO 2001/096295 (20.12.2001 Gazette 2001/51)**

(54) **2-CYANOPYRROLIDINE DERIVATIVES AND THEIR USE AS MEDICAMENTS**
2-CYANOPYRROLIDIN-DERIVATE UND IHRE VERWENDUNG ALS ARZNEIMITTEL
DERIVES DE 2-CYANOPYRROLIDINE ET LEUR UTILISATION COMME MEDICAMENTS

(84) Designated Contracting States:
**AT BE CH CY DE DK ES FI FR GB GR IE IT LI LU MC NL PT SE TR**
Designated Extension States:
**RO SI**

(30) Priority: **13.06.2000 US 592336**

(43) Date of publication of application:
**02.04.2003 Bulletin 2003/14**

(73) Proprietors:
- **NOVARTIS AG**
**4056 Basel (CH)**
Designated Contracting States:
**BE CH CY DE DK ES FI FR GB GR IE IT LI LU MC NL PT SE TR**
- **Novartis Pharma GmbH**
**1230 Wien (AT)**
Designated Contracting States:
**AT**

(72) Inventor: **VILLHAUER, Edwin, Bernard**
**Morristown, NJ 07960 (US)**

(74) Representative: **Gros, Florent et al**
**Novartis AG**
**4002 Basel (CH)**

(56) References cited:
**EP-A- 0 419 683** **WO-A-98/19998**
**US-A- 6 011 155**

- **T. E. HUGHES ET AL.: "(1-[[[2-[(5-Cyanopyridin-2-yl]amino]- acetyl]-2-cyano-(S)-pyrrolidine), a Slow-Binding Inhibitor of Dipeptidyl Peptidase IV" BIOCHEMISTRY, vol. 38, no. 36, 1999, pages 11597-11603, XP002184055**
- **BALKAN B ET AL: "INHIBITION OF DIPEPTIDYL PEPTIDASE IV WITH NVP-DPP728 INCREASES PLASMA GLP-1 (7-36 AMIDE) CONCENTRATIONS AND IMPROVES ORAL GLUCOSE TOLERANCE IN OBESE ZUCKER RATS" DIABETOLOGIA, BERLIN, DE, vol. 42, no. 11, November 1999 (1999-11), pages 1324-1331, XP000921066 ISSN: 0012-186X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 296 974 B1

## Description

The present invention relates to the area of dipeptidyl peptidase-IV inhibition and, more particularly, relates to certain N-(substituted glycyl)-2-cyanopyrrolidines, pharmaceutical compositions containing said compounds, and the use of said compounds in inhibiting dipeptidyl peptidase-IV.

Dipeptidyl peptidase-IV (DPP-IV) is a serine protease which cleaves N-terminal dipeptides from a peptide chain containing, preferably, a proline residue in the penultimate position. Although the biological role of DPP-IV in mammalian systems has not been completely established, it is believed to play an important role in neuropeptide metabolism, T-cell activation, attachment of cancer cells to the endothelium and the entry of HIV into lymphoid cells.

Likewise, it was discovered that DPP-IV is responsible for inactivating glucagon-like peptide-1 (GLP-1). Since GLP-1 is a major stimulator of pancreatic insulin secretion and has direct beneficial effects on glucose disposal, DPP-IV inhibition appears to represent an attractive approach e.g. for treating non-insulin-dependent diabetes mellitus (NIDDM).

The present invention provides new DPP-IV inhibitors which are effective e.g. in treating conditions mediated by DPP-IV inhibition, pharmaceutical compositions e.g. useful in inhibiting DPP-IV and a method of inhibiting DPP-IV.

Prior art compounds having related structures were already described in the patent application WO 98 19998 and in the patent US A 6 011 155.

The present invention provides compounds of formula I:

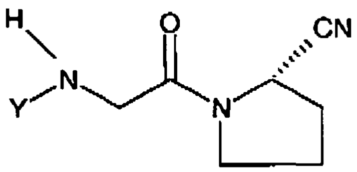

**(I)**

where Y is selected from the group consisting of:

i) a group of the formula

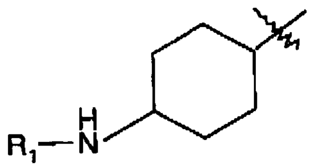

,

where $R_1$

is an unsubstituted pyridine, pyrimidine or phenyl ring; a pyridine, pyrimidine or phenyl ring which is mono- or independently di-substituted by halo, trifluoromethyl, cyano, nitro or $C_{1-6}$alkyl; an unsubstituted phenylsulfonyl group; a phenylsulfonyl group which is mono- or di-substituted on the phenyl ring by halo, trifluoromethyl, cyano, nitro or $C_{1-6}$alkyl; unsubstituted benzoyl; a benzoyl group which is mono- or di-substituted by halo or $C_{1-6}$alkyl; $C_1$-$_6$alkylcarbonyl; thienyl sulfonyl; unsubstituted benzothiazole; or a benzothiazole group which is substituted on the phenyl ring by halo or $C_{1-6}$alkyl;

ii) a group of the formula

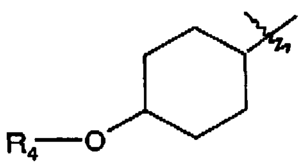

,

where $R_4$

is an unsubstituted phenyl ring; or a phenyl ring which is mono- or di-substituted by halo, trifluoromethyl, cyano, nitro or $C_{1-6}$alkyl; and iii)

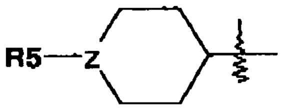

where $R_5$ is $C_{3-8}$-cycloalkyl-carbonyl, if Z is N; or $R_5$ is $C_{3-8}$cycloalkyl-carbonylamino, if Z is CH; or an acid addition salt thereof.

Preferred compounds are those of formula Ia:

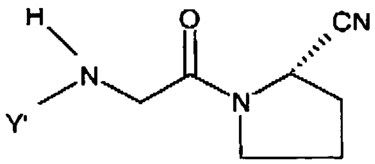

(Ia)

where Y' is selected from the group consisting of: i) a group of the formula

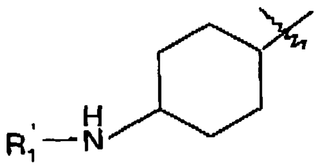

,

where $R_1$ is an unsubstituted pyridine, pyrimidine or phenyl ring; a pyridine, pyrimidine or phenyl ring which is monosubstituted by halo, trifluoromethyl, cyano, nitro or $C_{1-6}$-alkyl; an unsubstituted phenylsulfonyl group; a phenylsulfonyl group which is monosubstituted on the phenyl ring by halo, trifluoromethyl, cyano, nitro or $C_{1-6}$-alkyl; unsubstituted benzoyl; a benzoyl group which is monosubstituted by halo or $C_{1-6}$alkyl; $C_{1-6}$alkylcarbonyl: thienyl sulfonyl; unsubstituted benzothiazole; or a benzothiazole group which is substituted on the phenyl ring by halo or $C_{1-6}$alkyl; and
ii) a group of the formula

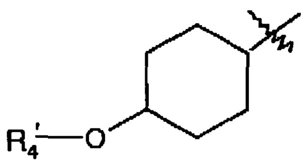

,

where $R_4$
is an unsubstituted phenyl ring; or a phenyl ring which is monosubstituted by halo, trifluoromethyl, cyano, nitro or $C_{1-6}$alkyl; or an acid addition salt thereof.

More preferred compounds are those of formula Ic:

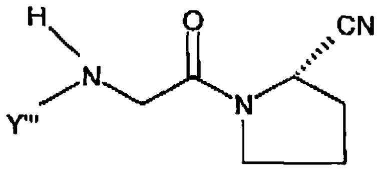

(Ic)

where Y''' is selected from the group consisting of: i) a group of the formula

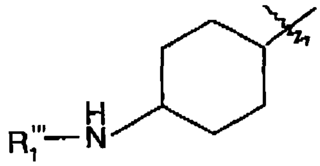

,

where $R_1'''$

is an unsubstituted pyridine, pyrimidine or phenyl ring; a pyridine, pyrimidine or phenyl ring which is monosubstituted by chloro, trifluoromethyl or cyano; an unsubstituted phenylsulfonyl group; a phenylsulfonyl group which is monosubstituted on the phenyl ring by chloro or trifluoromethyl; unsubstituted benzoyl; a benzoyl group which is monosubstituted by chloro; $C_{1-6}$alkylcarbonyl; thienyl sulfonyl; unsubstituted benzothiazole; or a benzothiazole group which is substituted on the phenyl ring by chloro; and
ii) a group of the formula

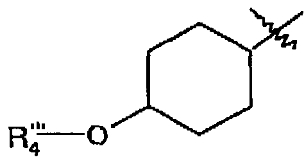

,

where $R_4'''$

is an unsubstituted phenyl ring; or a phenyl ring which is monosubstituted by chloro or trifluoromethyl;
or an acid addition salt thereof.

Compounds of formulae I, I a or I c, wherein Y represents a group of formula b); f); and g) [wherein Z is CH], are preferably in the trans orientation that is represented by formulae

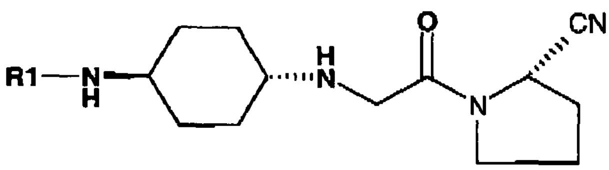

(I-b)

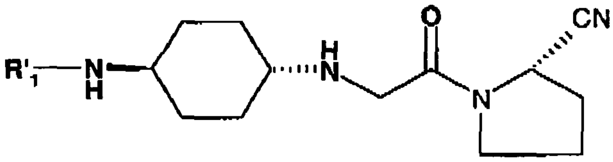

(I-b')

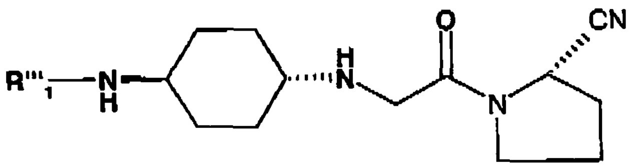

(I-b''')

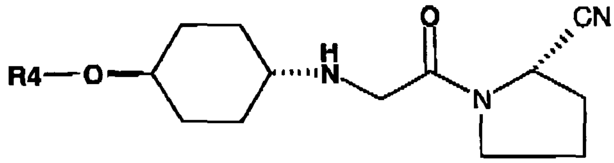

(I-f)

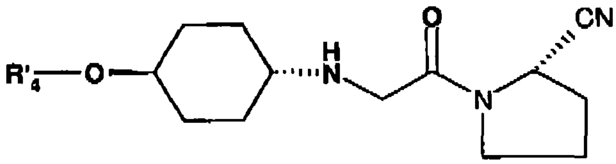

(I-f')

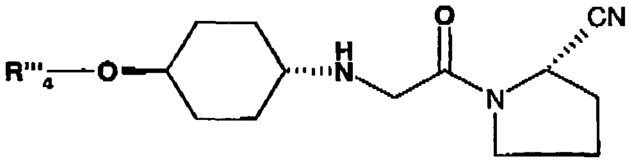

(I-f''')

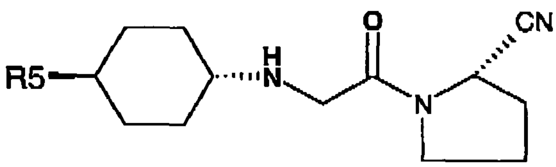

(I-g)

In another embodiment, the instant invention provides pharmaceutical compositions e.g. useful in inhibiting DPP-IV comprising a pharmaceutically acceptable carrier or diluent and a therapeutically effective amount of a compound of formula I above, or a pharmaceutically acceptable acid addition salt thereof, preferably a compound of formula Ia above, or a pharmaceutically acceptable acid addition salt thereof, more preferably a compound of formula Ib above, or a pharmaceutically acceptable acid addition salt thereof, and even more preferably a compound of formula Ic above, or a pharmaceutically acceptable acid addition salt thereof.

The present invention also relates to the use of a compound according to the instant invention or a pharmaceutically acceptable salt thereof e.g. for the manufacture of a medicament for the prevention or treatment of diseases or conditions associated with elevated levels of DPP-IV.

The compounds of formula I can exist in free form or in acid addition salt form. Pharmaceutically acceptable (i.e., non-toxic, physiologically acceptable) salts are preferred, although other salts are also useful, e.g., in isolating or purifying the compounds of this invention. Although the preferred acid addition salts are the hydrochlorides, salts of methanesulfonic, sulfuric, phosphoric, citric, lactic and acetic acid may also be utilized.

The compounds of the invention may exist in the form of optically active isomers or diastereoisomers and can be separated and recovered by conventional techniques, such as chromatography.

Listed below are definitions of various terms used to describe this invention. These definitions apply to the terms as they are used throughout this specification, unless otherwise limited in specific instances, either individually or as part of a larger group.

The term "halo" refers to chloro, fluoro, bromo or iodo.

The term "$C_{1-6}$alkyl" and the "$C_{1-6}$alkyl" portion of "di-$C_{1-6}$alkylaminocarbonyl" refers to straight or branched chain hydrocarbon groups having 1 to 6 carbon atoms, preferably 1 to 4 carbon atoms, more preferably 1 or 2 carbon atoms. Exemplary alkyl groups include methyl, ethyl, propyl, isopropyl, n-butyl, t-butyl, isobutyl, pentyl, hexyl and the like.

The "$C_{1-6}$alkyl" portion of "$C_{1-6}$alkylcarbonyl", in addition to the definition above, also refers to cyclic hydrocarbon groups, e.g. cyclopropyl, cyclobutyl, cyclopentyl and cyclohexyl.

The $C_{3-8}$ portion of $C_{3-8}$cycloalkyl-carbonyl refers to e.g. cyclopropyl, cyclobutyl, cyclopentyl and cyclohexyl.

The bond containing the wavy line signifies the point of attachment of the "Y" group to the glycyl-2-cyanopyrrolidine moiety-

The N-(substituted glycyl)-2-cyanopyrrolidines of the invention may be prepared, e.g., by a process which comprises coupling a reactive (2-cyanopyrrolidine)carbonylmethylene compound with an appropriate substituted amine. More particularly, the compounds of formula I may be prepared by reacting a compound of formula II

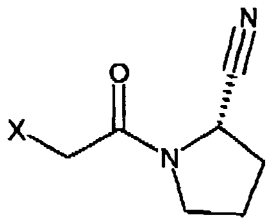

II

where X is a reactive group (preferably a halogen group such as chlorine, bromine or iodine, more preferably chlorine) with a compound of formula III

$$Y\text{-}NH_2 \qquad III$$

where Y is as defined above, and recovering the resultant compound of formula I in free form or in acid addition salt form-

The coupling may be effected by reacting the compound of formula II with 1 to 3 equivalents, preferably 3 equivalents, of a primary amine compound of formula III. The reaction is conveniently conducted in the presence of an inert, organic solvent, preferably a chlorinated, aliphatic hydrocarbon such as methylene chloride or a cyclic ether such as tetrahydrofuran, at a temperature of from about 0º to about 35ºC., preferably from about 0º to about 25ºC.

The compounds of the invention may be isolated from the reaction mixture and purified in conventional manner, e.g., by chromatography.

The starting compounds of formula II may be prepared by the following two-step reaction:

STEP 1 STEP 2

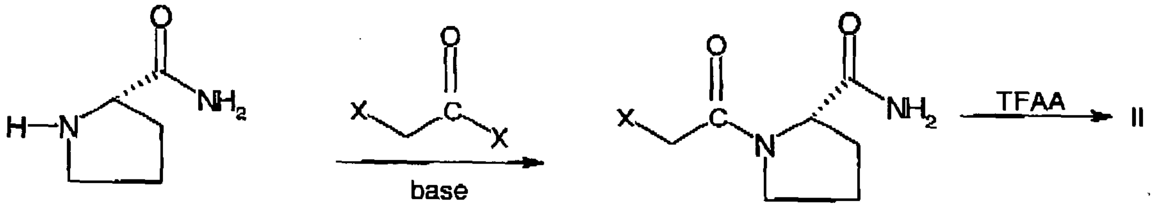


IV V

where X is as defined above.

Step 1 involves the reaction of the L-prolinamide compound of formula IV with a slight molar excess of a haloacetylhalide such a chloroacetylchloride or bromoacetylbromide and a base, e.g., an inorganic base such as potassium carbonate or an organic base such as triethylamine. The reaction is conveniently conducted in the presence of an inert, organic solvent, preferably a cyclic ether such as tetrahydrofuran or a chlorinated aliphatic hydrocarbon such as methylene chloride at a temperature of from about 0º to about 25ºC., preferably from about 0º to about 15ºC.

Step 2 concerns the dehydration of the compound prepared in Step 1, i.e, a compound of formula V, with 1 to 2 equivalents of trifluoroacetic anhydride (TFAA) to obtain a compound of formula II. The dehydration is conveniently conducted in the presence of an inert, organic solvent, preferably a cyclic ether such as tetrahydrofuran or a chlorinated, aliphatic hydrocarbon such as methylene chloride, at a temperature of from about 0º to about 25ºC., preferably from about 0º to about 15ºC.

Insofar as their preparation is not particularly described herein, the primary amine compounds of formula III are either known or may be prepared from known compounds in a known manner or analogously to known methods or analogously to methods described in the Examples. For example, the amine compounds of formula III may be prepared by reacting excess 1,2-diamino-2-methylpropane with the appropriate chloropyridine, chloropyrimidine, acid chloride, carbamoyl chloride or sulfonyl chloride. Thus, 2-[(5-chloro-2-pyridinyl)amino]-1,1-dimethylamine can be prepared by refluxing 2,5-dichloropyridine in excess 1,2-diamino-2-methylpropane for a period of between 2 and 12 hours. The following amines can be prepared in a similar fashion: a) 2-[(5-cyano-2-pyridinyl)amino]-1,1-dimethylethylamine from 5-cyano-2-chloropyridine, b) 2-[(5-trifluorometyl-2-pyridinyl)amino]-1,1 - dimethylethylamine from 5-trifluoromethyl-2-chloropyridine, c) 2-[(3-chloro-2-pyridinyl)amino]-1,1-dimethylethylamine from 2,3-dichloropyridine, d) 2-[(3,5-dichloro-2-pyridinyl)amino]-1,1-dimethylethylamine from 2,3,5-trichloropyridine, and e) 2-[(3-trifluoromethyl-2-pyridinyl)amino]-1,1-dimethylethylamine from 2-chloro-3-trifluoromethyl pyridine. The following amines can be prepared in a similar fashion at room temperature or lower, in the presence of an organic solvent, such as tetrahydrofuran and a base, such as potassium carbonate: a) 2-[(4-methylbenzoyl)amino]-1,1-dimethylethylamine from p-toluoyl chloride, b) 2-[(4-trifluoromethyl-2-pyridinyl)amino]-1, 1-dimethylethylamine from 2-chloro-4-(trifluoromethyl)pyridine, c) 2-[(2,2-dimethyl-1-oxopropyl)amino]-1,1-dimethylethyl]amine from trimethylacetyl chloride, d) 2-[(4-chlorobenzoyl)amino]-1,1-dimethylethylamine from 4-chlorobenzoyl chloride, e) 2-[[(diisopropylamino)carbonyl]amino]-1,1-dimethylethylamine from diisopropylcarbamylchloride, and f) 2-[[[(4-chlorophenyl)amino]carbonyl]amino]-1,1-dimethylethylamine from 4-chlorophenyl isocyanate. In addition, the amine compounds of formula III may be prepared by reacting excess trans-1,4-diaminocyclohexane with the appropriate chloropyridine, chloropyrimidine, acid chloride, carbamoyl chloride, chlorobenzothiazole or sulfonyl chloride. For example, 1-[4-[(5-cyano-2-pyridinyl)amino]cyclohexylamine can be prepared from 5-cyano-2-chloropyridine and two equivalents of 1,4-diaminohexane at room temperature in the presence of an organic solvent, such as dioxane and a base, such as potassium carbonate, for a period of between 2 and 48 hours. The following amines can be prepared in a similar fashion: a) 1-[4-[(phenylsulfonyl)amino]cyclohexyl]amine from phenylsulfonyl chloride, b) 1-[4-(benzoylamino)cyclohexyl]amine from benzoylchloride, c) 1-[4-[[(4-trifluoromethyl)-2-pyrimidinyl]amino]cyclohexyl] amine from 2-chloro-4-(trifluoromethyl)pyrimidine, d) 1-[4-[[(3-trifluoromethyl)-2-pyridinyl)amino]cyclohexyl]amine from 3-trifluoromethyl-2-chloropyridine, e) 1-[[4-[(4-chlorophenyl)sulfonyl]amino]cyclohexyl]amine from 4-chlorobenzenesulfonyl chloride, f) 1-[4-[(5-trifluoromethyl-2-pyridinyl)amino]cyclohexyl]amine from 5-trifluoromethyl-2-chloropyridine, g) 1-[4-[(2-chloro-4-pyrimidinyl)amino]cyclohexyl]amine from 2,4 dichloropyrimidine, h) 1-[4-[(4-chlorobenzoyl)amino]cyclohexyl]amine from 4-chlorobenzoyl chloride, i) 1-[4-[(2,2-dimethyl-1-oxopropyl)amino]cyclohexyl]amine from trimethylacetyl chloride, j) 1-[4-[(2-benzothiazolyl)amino]cyclohexyl]amine from 2-chlorobenzothiazole in THF at reflux for 18 hr., k) 1-[4-[(4-cyanophenyl)amino]cyclohexyl]amine from 4-aminobenzonitrile in DMF at 100ºC for 48 hours, l) 1-[4-[(cy-

clohexylcarbonyl) amino]cyclohexyl]amine from cyclohexanecarbonyl chloride, m) 1-[4-[(5-chloro-2-benzothiazolyl)amino]cyclohexyl]amine from 5-chloro-2-mercaptobenzothiazole at > 200ºC for 1 hr in 1,4-diaminocyclohexane as solvent, n) 1-[4-[(4-trifluoromethyl)phenyl]sulfonyl]amino]cyclohexyl]amine from 4-(trifluoromethyl)benzenesulfonyl chloride, and o) 1-[4-[[(2-thienyl)sulfonyl]amino]cyclohexyl]amine from 2-(thienyl)sulfonyl chloride. Moreover, the amine compounds of formula III may be prepared by reacting trans-4-aminocyclohexanol with the appropriate chloropyridine, chloropyrimidine, acid chloride, carbamoyl chloride, chlorobenzothiazole or sulfonyl chloride. For example, 1-[4-[4-(trifluoromethyl) phenoxy]cyclohexyl]amine can be prepared by slowly adding 4-fluorobenzotrifluoride (1.25 equivalents) to a suspension of sodium hydride (3.00 equivalents) and *trans*-4-aminocyclohexanol (1.00 equivalent) in DMF. The desired amine is obtained after stirring for three hours at 60ºC and then at room temperature for 18 hours. The following amines can be prepared in a similar fashion: a) 1-[4-[4-(chlorophenoxy)]cyclohexyl]amine from 1-chloro-4-fluorobenzene, b) 1-[4-[(3-trifluoromethyl)phenoxy]cyclohexyl]amine from 1-fluoro-3-trifluoromethylbenzene, and c) 1-[4-(3-chlorophenoxy)cyclohexyl]amine from 1-chloro-3-fluorobenzene. Furthermore, the amine compounds of formula III may be prepared by reacting *tert*-butyl-4-piperidylcarbamate with isocyanates and carbamyl chlorides followed by *tert*-butylcarbamate deprotection. For example, 1-[1-[[(4-chlorophenyl)amino]carbonyl]-4-piperidinyl]amine, monohydrochloride can be prepared with the addition of 4-chlorophenyl isocyanate (1.00 equivalent) to a solution of tert-butyl-4-piperidylcarbamate (1.00 equivalent) in tetrahydrofuran followed by stirring at ice water temperature for two hours, followed by deprotection of the resulting urea (hydrogen chloride in ethyl acetate). 1-[1-[(diisopropylamino)carbonyl]-4-piperidinyl]amine can be prepared in a similar fashion from diisopropylcarbamyl chloride. Still further, the amine compounds of formula III may be prepared from tert-butyl-4-piperidylcarbamate to provide 1-[1-[4-(4-Z-phenyl)-2-thiazolyl]-4-piperidinyl]amines where Z is either H, Cl or methoxy. For example, 1-[1-[4-(4-methoxyphenyl)-2-thiazolyl]-4-piperidinyl]amine, monohydride can be prepared with the addition of benzoyl isothiocyanate (1.00 equivalent) to a solution of tert-butyl-4-piperidylcarbamate (1.00 equivalent) in tetrahydrofuran followed by stirring at room temperature for two hours. Hydrolysis of the resulting benzoyl isothiocyanate ($K_2CO_3/H_2O$, reflux for 24 h) provides the thiourea which is reacted with 1.00 equivalent of 2-bromo-4'-methoxyacetophenone (EtOH, $NEt_3$ at reflux for 2 hours). Deprotection of the t-butylcarbamate protecting group (hydrogen chloride in ethyl acetate) provides the target amine monohydrochloride.
The following amines can be prepared in a similar fashion: a) 1-[1-(4-phenyl-2-thiazolyl)-4-piperidinyl]amine from 2-bromoacetophenone, and b) 1-[1-[4-(4-chlorophenyl)-2-thiazolyl]-4-piperidinyl)amine from 2-bromo-4'-chloroacetophenone.

The compounds of formula I having basic groups can be converted into acid addition salts, especially pharmaceutically acceptable acid addition salts. For example, the free base of a compound of formula I can be reacted with hydrochloric acid in gaseous form to form the corresponding mono- and di-hydrochloride salt forms, whereas reacting the free base with methanesulfonic acid forms the corresponding mesylate salt form. All pharmaceutically acceptable acid addition salt forms of the compounds of formula I are intended to be embraced by the scope of this invention.

In view of the close relationship between the free compounds and the compounds in the form of their salts, whenever a compound is referred to in this context, a corresponding salt is also intended, provided such is possible or appropriate under the circumstances.

The compounds, including their salts, can also be obtained in the form of their hydrates, or include other solvents used for their crystallization.

As indicated above, all of the compounds of formula I, and their corresponding pharmaceutically acceptable acid addition salts, are useful in inhibiting DPP-IV. The ability of the compounds of formula I, and their corresponding pharmaceutically acceptable acid addition salts, to inhibit OPP-IV may be demonstrated employing the Caco-2 DPP-IV Assay which measures the ability of test compounds to inhibit DPP-IV activity from human colonic carcinoma cell extracts. The human colonic carcinoma cell line Caco-2 was obtained from the American Type Culture Collection (ATCC HTB 37). Differentiation of the cells to induce DPP-IV expression was accomplished as described by Reisher, et al. in an article entitled "Increased expression of intestinal cell line Caco-2" in Proc. Natl. Acad. Sci., Vol. 90, pgs. 5757-5761 (1993). Cell extract is prepared from cells solubilized in 10mM Tris HCl, 0.15 M NaCl, 0.04 t.i.u.aprotinin, 0.5% nonidet-P40, pH 8.0, which is centrifuged at 35,000 g for 30 min. at 4ºC. to remove cell debris. The assay is conducted by adding 20 μg solubilized Caco-2 protein, diluted to a final volume of 125 μl in assay buffer (25 mM Tris HCl pH 7.4, 140mM NaCl, 10 mM KCl, 1% bovine serum albumin) to microtiter plate wells. After a 60 min. incubation at room temperature, the reaction is initiated by adding 25 μl of 1 mM substrate (H-Alanine-Proline-pNA; pNA is p-nitroaniline). The reaction is carried out at room temperature for 10 minutes after which time a 19 μl volume of 25% glacial acetic acid is added to stop the reaction. Test compounds are typically added as 30 μl additions and the assay buffer volume is reduced to 95 μl. A standard curve of free p-nitroaniline is generated using 0-500 μM solutions of free pNA in assay buffer. The curve generated is linear and is used for interpolation of substrate consumption (catalytic activity in nmoles substrate cleaved /min). The endpoint is determined by measuring absorbance at 405 nm in a Molecular Devices UV Max microtiter plate reader.

The potency of the test compounds as DPP-IV inhibitors, expressed as $IC_{50}$, is calculated from 8-point, dose-response curves using a 4-parameter logistic function.

The following $IC_{50}$'s were obtained:

| Compound | Caco-2 DPP-IV (nM) |
|---|---|
| Ex. 1 | 2 |
| Ex. 2L | 3 |
| Ex. 2M | 34 |
| Ex. 2N | 5 |
| Ex. 20 | 8 |
| Ex.2P | 15 |
| Ex. 2Q | 30 |
| Ex. 2R | 4 |
| Ex. 2S | 3 |
| Ex. 2T | 31 |
| Ex. 2U | 39 |
| Ex.2V | 9 |
| Ex. 2W | 13 |
| Ex. 2X | 22 |
| Ex. 2Y | 12 |
| Ex. 2Z | 66 |
| Ex. 2AA | 56 |
| Ex. 2EE | 23 |
| Ex. 2FF | 15 |
| Ex. 2GG | 22 |
| Ex.2HH | 18 |
| Ex. 3 | 124 |
| Ex. 4A | 24 |
| Ex. 5 | 36 |

The ability of the compounds of formula I, and their corresponding pharmaceutically acceptable acid addition salts, to inhibit DPP-IV may also be demonstrated by measuring the effects of test compounds on DPP-IV activity in human and rat plasma employing a modified version of the assay described by Kubota, et al. in an article entitled "Involvement of dipeptidylpeptidase IV in an in vivo immune response" in Clin. Exp. Immunol., Vol. 89, pgs. 192-197 (1992). Briefly, 5 μl of plasma are added to 96-well flat-bottom micortiter plates (Falcon), followed by the addition of 5 μl of 80 mM $MgCl_2$ in incubation buffer (25 mMHEPES, 140 mM NaCl, 1% RIA-grade BSA, pH 7.8). After a 60 min. incubation at room temperature, the reaction is initiated by the addition of 10 μl of incubation buffer containing 0.1 mM substrate (H-Glycine-Proline-AMC; AMC is 7-amino-4-methylcoumarin). The plates are covered with aluminum foil (or kept in the dark) and incubated at room temperature for 20 min. After the 20 min. reaction, florescence is measured using a CytoFluor 2350 fluorimeter (Excitation 380 mn Emission 460nm; sensitivity setting 4). Test compounds are typically added as 2 μl additions and the assay buffer volume is reduced to 13 μl. A fluorescence-concentration curve of free AMC is generated using 0-50 μM solutions of AMC in assay buffer. The curve generated is linear and is used for interpolation of substrate consumption (catalytic activity in nmoles substrate cleaved/min). As with the previous assay, the potency of the test compounds as DPP-IV inhibitors, expressed as $IC_{50}$, is calculated from 8-point, dose-response curves using a 4 parameter logistic function.

The following $IC_{50}$'s were obtained:

| Compound | human plasma DPP-IV (nM) | rat plasma DPP-IV (nM) |
|---|---|---|
| Ex.1 | 30 | 6 |
| Ex. 2L | 9 | 4 |
| Ex. 2M | 10 | 6 |
| Ex. 2N | 10 | 5 |
| Ex. 2O | 8 | 9 |
| Ex.2P | 16 | 11 |
| Hex. 20 | 38 | 38 |
| Ex. 2R | 23 | 15 |
| Ex. 2S | 4 | 2 |
| Ex. 2T | 26 | 37 |
| Ex. 2U | 27 | 14 |
| Ex. 2V | 28 | 11 |
| Ex. 2W | 19 | 7 |
| Ex. 2X | 47 | 20 |
| Ex. 2Y | 124 | 37 |
| Ex. 2Z | 122 | 88 |
| Ex. 2AA | 33 | 16 |
| Ex. 2EE | 150 | 46 |
| Ex. 2FF | 94 | 42 |
| Ex. 2GG | 20 | 10 |
| Ex. 2HH | 18 | 10 |
| Ex. 3 | 204 | 107 |
| Ex. 4A | 48 | 20 |
| Ex. 5 | 5 | 4 |

In view of their ability to inhibit DPP-IV, the compounds of formula I, and their corresponding pharmaceutically acceptable acid addition salts, are useful in treating conditions mediated by DPP-IV inhibition. Based on the above and findings in the literature, it is expected that the compounds disclosed herein are useful in the treatment of conditions such as non-insulin-dependent diabetes mellitus, arthritis, obesity, allograft transplantation and calcitonin-osteoporosis. In addition, based on the roles of glucagon-like peptides (such as GLP-1 and GLP-2) and their association with DPP-IV inhibition, it is expected that the compounds disclosed herein are useful for example, to produce a sedative or anxiolytic effect, or to attenuate post-surgical catabolic changes and hormonal responses to stress, or to reduce mortality and morbidity after myocardial infarction, or in the treatment of conditions related to the above effects which may be mediated by GLP-1 and/or GLP-2 levels.

More specifically, for example, the compounds of formula 1, and their corresponding pharmaceutically acceptable acid addition salts, improve early insulin response to an oral glucose challenge and, therefore, are useful in treating non-insulin-dependent diabetes mellitus. The ability of the compounds of formula I, and their corresponding pharmaceutically acceptable acid addition salts, to improve early insulin response to an oral glucose challenge may be measured in insulin resistant rats according to the following method:

Male Sprague-Dawley rats that had been fed a high fat diet (saturated fat = 57% calories) for 2-3 weeks were fasted for approximately 2 hours on the day of testing, divided into groups of 7-10, and dosed orally with 10 μmol/kg of

test compound in carboxymethylcellulose. Each of the test compounds administered orally at 10 μmol/kg ten minutes prior to the administration of glucose (1 g/kg p.o.), led to a significant inhibition of plasma DPP-IV activity during the study. For example, the compound of Example 2N, administered orally at 10 μmol/kg (n=7-8) ten minutes prior to the administration of glucose (1 g/kg p.o.), led to an 80% inhibition of plasma DPP-IV activity during the study. Blood samples, obtained at various time-points from chronic jugular vein catheters, were analyzed for plasma glucose concentration. Data are expressed as % decrease of the area under the plasma glucose curve compared to vehicle-treated control animals. The following result was obtained:

| Compound | Decrease of plasma glucose excursion at 10 μmovkg (p=0.01) |
|---|---|
| Ex. 2N | 39% |

The precise dosage of the compounds of formula I, and their corresponding pharmaceutically acceptable acid addition salts, to be employed for treating conditions mediated by DPP-IV inhibition depends upon several factors, including the host, the nature and the severity of the condition being treated, the mode of administration and the particular compound employed. However, in general, conditions mediated by DPP-IV inhibition are effectively treated when a compound of formula I, or a corresponding pharmaceutically acceptable acid addition salt, is administered enterally, e.g., orally, or parenterally, e.g., intravenously, preferably orally, at a daily dosage of 0.002-5, preferably 0.02-2.5 mg/kg body weight or, for most larger primates, a daily dosage of 0.1-250, preferably 1-100 mg. A typical oral dosage unit is 0.01-0.75 mg/kg, one to three times a day. Usually, a small dose is administered initially and the dosage is gradually increased until the optimal dosage for the host under treatment is determined. The upper limit of dosage is that imposed by side effects and can be determined by trial for the host being treated.

The compounds of formula I, and their corresponding pharmaceutically acceptable acid addition salts, may be combined with one or more pharmaceutically acceptable carriers and, optionally, one or more other conventional pharmaceutical adjuvants and administered enterally, e.g., orally, in the form of tablets, capsules, caplets, etc or parenterally, e.g., intravenously, in the form of sterile injectable solutions or suspensions. The enteral and parenteral compositions may be prepared by conventional means.

The compounds of formula I, and their corresponding pharmaceutically acceptable acid addition salts, may be formulated into enteral and parenteral pharmaceutical compositions containing an amount of the active substance that is effective for treating conditions mediated by DPP-IV inhibition, such compositions in unit dosage form and such compositions comprising a pharmaceutically acceptable carrier.

The compounds of formula I (including those of each of the subscopes thereof and each of the examples) may be administered in enantiomerically pure form (e.g., ee>98%, preferably >99%) or together with the R enantiomer, e.g., in racemic form. The above dosage ranges are based on the compounds of formula I (excluding the amount of the R enantiomer).

The present invention furthermore refers to a combination, especially a combined preparation or pharmaceutical composition, respectively, comprising a compound of formula I or a pharmaceutically acceptable salt thereof and at least one different antidiabetic agent (e.g. one or two different antidiabetic agents) or a pharmaceutically acceptable salt thereof.

A suitable antidiabetic agent is e.g. selected from the group consisting of insulin signalling pathway modulators, like inhibitors of protein tyrosine phosphatases (PTPases), non-small molecule mimetic compounds and inhibitors of glutamine-fructose-6-phosphate amidotransferase (GFAT), compounds influencing a dysregulated hepatic glucose production, like inhibitors of glucose-6-phosphatase (G6Pase), inhibitors of fructose-1,6-bisphosphatase (F-1,6-BPase), inhibitors of glycogen phosphorylase (GP), glucagon receptor antagonists and inhibitors of phosphoenolpyruvate carboxykinase (PEPCK), pyruvate dehydrogenase kinase (PDHK) inhibitors, insulin sensitivity enhancers, insulin secretion enhancers, α-glucosidase inhibitors, inhibitors of gastric emptying, insulin, and $\alpha_2$-adrenergic antagonists for simultaneous, separate or sequential use.

Examples of "inhibitors of PTPase" include those disclosed in U.S. Patent No. 6,057,316, U.S. Patent No. 6,001,867, WO 99/58518, WO 99/58522, WO 99/46268, WO 99/46267, WO 99/46244, WO 99/46237, WO 99146236, WO 99115529 and by Poucheret et al in Mol. Cell Biochem. 1998, 188, 73-80.

Examples of "non-small molecule mimetic compounds" include those disclosed in Science 1999, 284; 974-97, especially L-783,281, and WO 99/58127, especially CLX-901.

Examples of "inhibitors of GFAT" include those disclosed in Mol. Cell. Endocrinol. 1997,135(1), 67-77.

The term "inhibitors of G6Pase" used herein means a compound or composition which reduces or inhibits hepatic gluconeogenesis by decreasing or inhibiting the activity of G6Pase. Examples of such compounds are disclosed in WO 00/14090 , WO99/40062, WO 98/40385, EP682024 and Diabetes 1998, 47,1630-1636-

The term "inhibitors of F-1,6-BPase" used herein means a compound or composition which reduces or inhibits hepatic gluconeogenesis by decreasing or inhibiting the activity of F-1,6-BPase. Examples of such compounds are

disclosed in WO 00/14095, WO 99/47549, WO 98/39344, WO 98/39343 and WO 98/39342.

The term "inhibitors of GP" used herein means a compound or composition which reduces or inhibits hepatic glycogenolysis by decreasing or inhibiting the activity of GP. Examples of such compounds are disclosed in EP 978279, US Patent No. 5998463, WO 99/26659, EP 846464, WO 97131901, WO 96/39384, WO9639385 and in particular CP-91149 as described in Proc. Natl. Acad Sci USA 1998, 95, 1776-1781.

The term "glucagon receptor antagonists" as used herein relates in particular to the compounds described in WO 98/04528, especially BAY27-9955, and those described in Bioorg Med. Chem. Lett 1992, 2, 915-918, especially CP-99,711, J. Med. Chem. 1998, 41, 5150-5157, especially NNC 92-1687, and J. Biol Chem. 1999, 274; 8694-8697, especially L-168,049 and compounds disclosed in US 5,880,139, WO 99101423, US 5,776,954, WO 98/22109, WO 98/22108, WO 98/21957 and WO 97/16442.

The term "inhibitors of PEPCK" used herein means a compound or composition which reduces or inhibits hepatic gluconeogenesis by decreasing or inhibiting the activity of PEPCK. Examples of such compounds are disclosed in U.S. Patent No. 6,030,837 and Mol. Biol. Diabetes 1994, 2, 283-99.

The term "PDHK inhibitors" as used herein means inhibitors of pyruvate dehydrogenase kinase and include those compounds disclosed by Aicher et al in J. Med. Chem. 42 (1999) 2741-2746.

The term "insulin sensitivity enhancer" used herein means any and all pharmacological active compounds that enhance the tissue sensitivity towards insulin. Insulin sensitivity enhancers include, e.g., inhibitors of GSK-3, retinoid X receptor (RXR) agonists, agonists of Beta-3 AR, agonists of UCPs, antidiabetic thiazolidinediones (glitazones), non-glitazone type PPARγ agonists, dual PPARγ / PPARα agonists, antidiabetic vanadium containing compounds and biguanides, e.g., metformin.

The insulin sensitivity enhancer is preferably selected from the group consisting of antidiabetic thiazolidinediones, antidiabetic vanadium containing compounds and metformin.

In one preferred embodiment, the insulin sensitivity enhancer is metformin.

Examples of "inhibitors of GSK-3" include those disclosed in WO 00/21927 and WO 97/41854.

By "RXR agonist" is meant a compound or composition which when combined with RXR homodimers or heterodimers increases the transcriptional regulation activity of RXR, as measured by an assay known to one skilled in the art, including the "co-transfection" or "cis-trans" assays described or disclosed in U.S. Pat. Nos. 4,981,784, 5,071,773, 5,298,429, 5,506,102, WO89/05355, WO91/06677, WO92/05447, WO93111235, WO95/18380, PCT/US93/04399, PCT/US94/03795 and CA 2,034,220. It includes compounds that preferentially activate RXR over RAR (i.e. RXR specific agonists), and compounds that activate both RXR and RAR (i.e. pan agonists). It also includes compounds that activate RXR in a certain cellular context but not others (i.e. partial agonists). Compounds disclosed or described in the following articles, patents and patent applications have RXR agonist activity: U.S. Pat. Nos. 5;399,586 and 5,466,861, WO96/05165, PCT/US95/16842, PCT/US95/16695, PCT/US93/10094, WO94/15901, PCT/US92/11214, WO93/11755, PCT/US93/10166, PCT/US93/10204, WO94/15902. PCT/US93/03944, WO93/21146, provisional applications 60,004,897 and 60,009,884, Boehm, et al. J. Med. Chem. 38(16):3146-3155,1994. Boehm, et al. J. Med. Chem. 37(18): 2930-2941, 1994, Antras et al., J. Biol. Chem. 266:1157-1161 (1991), Salazar-Olivo et al., Biochem. Biophys. Res. Commun. 204:157-263 (1994) and Safanova, Mol. Cell. Endocrin. 104:201-211 (1994). RXR specific agonists include LG 100268 (i.e. 2-[1-(3,5,5,8,8-pentamethyl-5.6,7,8-tetrahydro-2-naphthyl)-cyclopropyl]-py ridine-5-carboxylic acid) and LGD 1069 (i.e. 4-[(3,5,5,8,8-pentamethyl-5,6,7,8-tetrahydro-2-naphthyl)-2-carbonyl]-benzo ic acid), and analogs, derivatives and pharmaceutically acceptable salts thereof. The structures and syntheses of LG 100268 and LGD 1069 are disclosed in Boehm, et al. J. Med. Chem. 38(16): 3146-3155, 1994. Pan agonists include ALRT 1057 (i.e. 9-cis retinoic acid), and analogs, derivatives and pharmaceutically acceptable salts thereof.

Examples of "agonists of Beta-3 AR" include CL-316,243 (Lederle Laboratories) and those disclosed in WO 99/29672, WO 98/32753, WO 98/20005, WO 98/09625, WO 97/46556, WO 97/37646 and U.S. Patent No. 5,705,515.

The term "agonists of UCPs" used herein means agonists of UGP-1, preferably UCP-2 and even more preferably UCP-3. UCPs are disclosed in Vidal-Puig et al., Biochem. Biophys. Res. Commun., Vol. 235(1) pp. 79-82 (1997). Such agonists are a compound or composition which increases the activity of UCPs.

The antidiabetic thiazolidinedione (glitazone) is, for example, (S)-((3,4-dihydro-2-(phenyl-methyl)-2H-1-benzopyran-6-yl)methyl-thiazolidine-2,4-dione (englitazone), 5-{[4-(3-(5-methyl-2-phenyl-4-oxazolyl)-1-oxopropyl)-phenyl]-methyl}-thiazolidine-2,4-dione (darglitazone), 5-{[4-(1-methyl-cyclohexyl)methoxy}-phenyl]methyl}-thiazolidine-2,4-dione (ciglitazone), 5-{[4-(2-(1-indolyl)ethoxy)phenyl]methyl}-thiazolidine-2,4-dione (DRF2189), 5-(4-[2-(5-methyl-2-phenyl-4-oxazoly)-ethoxy)]benzyl}thiazolidine-2,4-dione (BM-13.1246), 5-(2-naphthylsulfonyl)-thiazolidine-2,4-dione (AY-31637), bis{4-[(2,4-dioxo-5-thiazolidinyl)-methyl]phenyl}methane (YM268). 5-{4-[2-{5-methyl-2-phenyl-4-oxazolyl)-2-hydroxyethoxy]-benzyl}-thiazolidine-2,4-dione (AD-5075), 5-[4-(1-phenyl-1-cyclopropanecarbonylamino)-benzyl]-thiazolidine-2,4-dione (DN-108) 5-{[4-(2-(2,3-dihydroindol-1-yl)ethoxy)phenylmethyl}-thiazolidine-2,4-dione, 5-[3-(4-chloro-phenyl])-2-propynyl]-5-phenylsulfonyl)thiazolidine-2,4-dione, 5-[3-(4-chlorophenyl])-2-propynyl]-5-(4-fluorophenylsulfonyl)thiazolidine-2,4-dione, 5-{[4-(2-(methyl-2-pyridinyl-amino)-ethoxy)phenyl]methyl}-thiazolidine-2,4-dione (rosiglitazone), 5-{[4-(2-(5-ethyl-2-pyridyl)ethoxy)phenyl]-methyl}thiazolidine-2.4-dione (pioglitazone), 5-{[4-((3,4-dihydro-6-hydroxy-2,5,7,8-tetramethyl-2H-1-benzopyran-2-yl)methoxy)-phenyl]-methyl}-thiazolidine-2,4-dione (troglitazone), 5-[6-(2-fluoro-benzyloxy)naphthalen-2-ylmethyl]-thiazolidine-2,4-dione (MCC555). 5-{[2-(2-naphthyl)-benzoxazol-5-yl]-methyl}thiazolidine-2,4-dione (T-174) and 5-(2,4-dioxothiazolidin-5-ylmethyl)-2-methoxy-N-(4-trifluoromethylbenzyl) benzamide (KRP297).

The glitazones 5-{[4-(2-{5-ethyl-2-pyridyl)ethoxy)phenyl]-methyl}thiazolidine-2,4-dione (pioglitazone, EP 0 193 256 A1), 5-{[4-(2-(methyl-2-pyridinyl-amino)-ethoxy)phenyl]methyl}thiazolidine-2,4-dione (rosiglitazone, EP 0 306 228 A1), 5-{[4-((3,4-dihydro-6-hydroxy-2,5,7,8-tetramethyl-2H-1-benzopyran-2-yl)methoxy)-phenyl]-methyllthiazolidine-2,4-dione (troglitazone, EP 0 139 421), (S)-((3,4-dihydro-2-(phenylmethyl)-2H-1-benzopyran-6-yl)methyl-thiazolidine-2,4-dione (englitazone, EP 0 207 605 B1), 5-(2,4-dioxothiazolidin-5-ylmethyl)-2-methoxy-N-(4-trifluoromethyl-benzyl)benzamide (KRP297, JP 10087641-A), 5-[6-(2-fluoro-benzyloxy)naphthalen-2-ylmethyl]thiazolidine-2,4-dione (MCC555, EP 0 604 983 B1), 5-{[4-(3-(5-methyl-2-phenyl-4-oxazolyl)-1-oxopropyl)-phenyl]-methyl}-thiazolidine-2,4-dione (darglitazone, EP 0 332 332), 5-(2-naphthylsulfonyl)-thiazolidine-2,4-dione (AY-31637, US 4,997,948), 5-{[4-(1-methyl-cyclohexyl)methoxy)-phenyl]methyl}-thiazolidine-2,4-dione (ciglitazone, US 4,287,200) are in each case generically and specifically disclosed in the documents cited in brackets beyond each substance, in each case in particular in the compound claims and the final products of the working examples. The preparation of DRF2189 and of 5-{[4-(2-(2,3-dihydroindol-1-yl)ethoxy)phenyl]methyl}-thiazolidine-2,4-dione is described in B.B. Lohray et al., J. Med. Chem. 1998, 41, 1619-1630; Examples 2d and 3g on pages 1627 and 1628. The preparation of 5-[3-(4-chlorophenyl])-2-propynyl]-5-phenylsulfonyl)-thiazolidine-2,4-dione and the other compounds in which A is phenylethynyl mentioned herein can be carried out according to the methods described in J. Wrobel et al., J. Med. Chem. 1998, 41, 1084-1091.

In particular, MCC555 can be formulated as disclosed on page 49, lines 30 to 45, of EP 0 604 983 B1; englitazone as disclosed from page 6, line 52, to page 7, line 6, or analogous to Examples 27 or 28 on page 24 of EP 0 207 605 B1; and darglitazone and 5-{4-[2-(5-methyl-2-phenyl-4-oxazolyl)-ethoxy)]benzyl}-thiazolidine-2,4-dione (BM-13.1246) can be formulated as disclosed on page 8, line 42 to line 54 of EP 0 332 332 B1. AY-31637 can be administered as disclosed in column 4, lines 32 to 51 of US 4,997,948 and rosiglitazone as disclosed on page 9, lines 32 to 40 of EP 0 306 228 A1, the latter preferably as its maleate salt. Rosiglitazone can be administered in the form as it is marketed e.g. under the trademark AVANDIA™. Troglitazone can be administered in the form as it is marketed e.g. under the trademarks ReZulin™, PRELAY™, ROMOZIN™ (in the United Kingdom) or NOSCAL™ (in Japan). Pioglitazone can be administered as disclosed in Example 2 of EP 0 193 256 A1, preferably in the form of the monohydrochloride salt. Corresponding to the needs of the single patient it can be possible to administer pioglitazone in the form as it is marketed e.g. under the trademark ACTOS™. Ciglitazone can, for example, be formulated as disclosed in Example 13 of US 4,287,200.

Non-glitazone type PPARγ agonists are especially N-(2-benzoylphenyl)-L-tyrosine analogues, e.g. GI-262570, and JTT501.

The term "dual PPARγ / PPARα agonists" as used herein means compounds which are at the same time PPARγ and PPARα agonists. Preferred dual PPARγ / PPARα agonists are especially those ω-[(oxoquinazolinylalkoxy)phenyl] alkanoates and analogs thereof , very especially the compound DRF-554158, described in WO 99/08501 and the compound NC-2100 described by Fukui in Diabetes 2000, 49(5), 759-767.

Preferably, the antidiabetic vanadium containing compound is a physiologically tolerable vanadium complex of a bidentate monoprotic chelant, wherein said chelant is an α-hydroxypyrone or α-hydroxypyridinone, especially those disclosed in the Examples of US 5,866,563, or a pharmaceutical acceptable salt thereof.

The preparation of metformin (dimethyldiguanide) and its hydrochloride salt is state of the art and was disclosed first by Emil A. Wemer and James Bell, J. Chem. Soc-121, 1922, 1790-1794. Metformin. can be administered e.g. in the form as marketed under the trademarks GLUCOPHAGE™.

Insulin secretion enhancers are pharmacological active compounds having the property to promote secretion of insulin from pancreatic β cells. Examples for insulin secretion enhancers include glucagon receptor antagonists (see above), sulphonyl urea derivatives, incretin hormones, especially glucagon-like peptide-1 (GLP-1) or GLP-1 agonists, β-cell imidazoline receptor antagonists, and short-acting insulin secretagogues, like antidiabetic phenylacetic acid derivatives, antidiabetic D-phenylalanine derivatives and BTS 67582 described by T. Page et al in Br. J. Pharmacol. 1997, 122, 1464-1468-

The sulphonyl urea derivative is, for example, glisoxepid, glyburide, glibenclamide, acetohexamide, chloropropamide, glibomuride, tolbutamide, tolazamide, glipizide, carbutamide, gliquidone, glyhexamide, phenbutamide or tolcyclamide; and preferably glimepiride or gliclazide. Tolbutamide, glibenclamide, gliclazide, glibomuride, gliquidone, glisoxepid and glimepiride can be administered e.g. in the form as they are marketed under the trademarks RASTINON HOECHST™, AZUGLUCON™, DIAMICRON™, GLUBORID™, GLURENORM™, PRO-DIABAN™ and AMARYL™, respectively.

GLP-1 is a insulinotropic proteine which was described, e.g., by W.E. Schmidt et al. in Diabetologia 28, 1985, 704-707 and in US 5,705,483. The term "GLP-1 agonists" used herein means variants and analogs of GLP-1(7-36)$NH_2$ which are disclosed in particular in US 5,120,712, US 5,118666, US 5,512,549, WO 91/11457 and by C. Orskov et al in J. Biol. Chem. 264 (1989) 12826. The term "GLP-1 agonists" comprises especially compounds like GLP-1 (7-37), in

which compound the carboxy-terminal amide functionality of $Arg^{36}$ is displaced with Gly at the $37^{th}$ position of the GLP-1(7-36)$NH_2$ molecule and variants and analogs thereof including $GLN^9$-GLP-1(7-37), D-$GLN^9$-GLP-1(7-37), acetyl $LYS^9$-GLP-1(7-37), $LYS^{18}$-GLP-1(7-37) and, in particular, GLP-1 (7-37)OH, $VAL^8$-GLP-1 (7-37), $GLY^8$-GLP-1 (7-37), $THR^8$-GLP-1 (7-37), $MET^8$-GLP-1 (7-37) and 4-imidazopropionyl-GLP-1. Special preference is also given to the GLP agonist analog exendin-4, described by Greig et al in Diabetologia 1999, 42, 45-50.

The term "β-cell imidazoline receptor antagonists" as used herein means compounds as those described in WO 00/78726 and by Wang et al in J. Pharmacol. Exp. Ther. 1996; 278; 82-89, e.g. PMS 812.

The antidiabetic phenylacetic acid derivative is preferably repaglinide or a pharmaceutically acceptable salt thereof.

Most preferably, the antidiabetic D-phenylalanine derivative is nateglinide or a pharmaceutically acceptable salt thereof.

Nateglinide (N-[(trans-4-isopropylcyclohexyl)-carbonyl]-D-phenylalanine, EP 196222 and EP 526171) and repaglinide ((S)-2-ethoxy--4-{2-[[3-methyl-1-[2-(1-piperidinyl)phenyl]-butyl]amino]-2-oxoethyl}benzoic acid, EP 0 147 850 A2, in particular Example 11 on page 61, and EP 0 207 331 A1) are in each case generically and specifically disclosed in the documents cited in brackets beyond each substance, in each case in particular in the compound claims and the final products of the working examples. The term nateglinide as used herein comprises crystal modifications (polymorphs) such as those disclosed in EP 0526171 B1 or US 5,488,510, respectively, especially the subject matter of claims 8 to 10 as well as the corresponding references to the B-type crystal modification. Preferably, in the present invention the B- or H-type, more preferably the H-type, is used. Repaglinde can be administered in the form as it is marketed e.g. under the trademark NovoNorm™. Nateglinide can be administered in the form as it is marketed e.g. under the trademark STARLIX™.

α-Glucosidase inhibitors are pharmacological active compounds which inhibit small intestinal α-glucosidase enzymes which break down non-adsorbable complex carbohydrates into absorbable monosaccharides. Examples for such compounds are acarbose, N-(1,3-dihydroxy-2-propyl)valiolamine (voglibose) and the 1-deoxynojirimycin derivative miglitol. Acarbose is 4",6"-dideoxy-4"-[(1S)-(1,4,6/5)-4,5,6-trihydroxy-3-hydroxymethyl-2-cyclo-hexenylamino}maltotriose. The structure of acarbose can as well be described as O-4,6-dideoxy-4-([1S,4R,5S,6S]-4,5,6-trihydroxy-3-(hydroxymethyl)-2-cyclohexen-1-yl]-amino}-α-D-glucopyranosyl-(1→4)-O-α-D-glucopyranosyl-(1→4)-D-glucopyranose. Acarbose (US 4,062,950 and EP 0 226 121), is generically and specifically disclosed in the documents cited in brackets, in particular in the compound claims and the final products of the working examples. Corresponding to the needs of the single patient it can be possible to administer acarbose in the form as it is marketed e.g. under the trademark GLUCOBAY™. Miglitol can be administered in the form as it is marketed e.g. under the trademark DIASTABOL 50™

The α-glucosidase inhibitor is preferably selected from the group consisting of acarbose, voglibose and miglitol.

Examples of "inhibitors of gastric emptying" other than GLP-1 include those disclosed in J. Clin. Endocrinol. Metab. 2000, 85(3), 1043-1048, especially CCK-8, and in Diabetes Care 1998; 21; 897-893, especially Amylin and analogs thereof, e.g. Pramlintide. Amylin is also described e.g. by O.G. Kolterman et al. in Diabetologia 39, 1996, 492-499.

Examples of "$\alpha_2$-adrenergic antagonists" include midaglizole described in Diabetes 36, 1987. 216-220.

Comprised are likewise the corresponding stereoisomers as well as the corresponding polymorphs, e.g. crystal modifications, which are disclosed in the cited patent documents.

In a very preferred embodiment of the invention, the further antidiabetic compound is selected from the group consisting of nateglinide, repaglinide, metformin, rosiglitazone, pioglitazone, troglitazone, glisoxepid, glyburide, glibenclamide, acetohexamide, chloropropamide, glibomuride, tolbutamide, tolazamide, glipizide, carbutamide, gliquidone, glyhexamide, phenbutamide, tolcyclamide, glimepiride and gliclazide, or the pharmaceutically acceptable salt of such a compound. Most preferred is nateglinide, repaglinide or metformin, respectively, furthermore, pioglitazone, rosiglitazone or troglitazone respectively.

The structure of the active agents identified by code nos., generic or trade names may be taken from the actual edition of the standard compendium "The Merck Index" or from databases, e.g. Patents International (e.g. IMS World Publications). Any person skilled in the art is fully enabled to identify the active agents and, based on these references, likewise enabled to manufacture and test the pharmaceutical indications and properties in standard test models, both *in vitro* and in *vivo.*

The combinations according to the present invention can used especially in the prevention, delay of progression or treatment of conditions mediated by dipeptidylpeptidase - IV (DPP-IV), in particular diabetes, more particular type 2 diabetes mellitus, conditions of impaired glucose tolerance (IGT), conditions of impaired fasting plasma glucose, metabolic acidosis, ketosis, arthritis, obesity and osteoporosis; for the prevention, delay of progression or treatment of such conditions; the use of such combination for the cosmetic treatment of a mammal in order to effect a cosmetically beneficial loss of body weight.

The person skilled in the pertinent art is fully enabled to select a relevant animal test model to prove the hereinbefore and hereinafter indicated therapeutic indications and beneficial effects.

The invention furthermore relates to a commercial package comprising a compound according to the present

invention or a combination according to the present invention together with instructions for simultaneous, separate or sequential use.

The following examples show representative compounds encompassed by this invention and their synthesis. However, it should be clearly understood that they are for purposes of illustration only.

## EXAMPLE 1

1-[[[2-[(5-Chloro-2-pyridinyl)amino]-1,1-dimethylethyl]amino]acetyl]-2-cyano-(S)-pyrrolidine, monohydrochloride

A. 1-Chloroacetyl-2-(S)-cyanopyrrolidine

To a mechanically stirred solution of 20.0 g (180.0 mmol) of chloroacetylchloride and 97 g (0.70 mmol) of potassium carbonate in 150 mL of tetrahydrofuran is added a solution of L-prolinamide 20.0 g (180.0 mmol) in 500 mL of tetrahydrofuran in a dropwise fashion over 45 minutes. This reaction is then mechanically stirred for an additional two hours at room temperature. The reaction is then filtered to remove potassium salts and the filtrate is dried over $Na_2SO_4$. The $Na_2SO_4$ is then removed via filtration and to this colorless filtrate is added trifluoroacetic anhydride (25.0 mL, 0.180 mmol) in one portion. The reaction is then magnetically stirred for 1 hour at room temperature and the resulting clear yellow/orange solution is concentrated via rotovap. The excess trifluoroacetic anhydride is removed by adding ethyl acetate to the concentrated oil and reconcentrating via rotovap. This removing operation is performed three times.

The resulting oil is partitioned between ethyl acetate and water. The product is then extracted into the ethyl acetate and the aqueous layer is then washed twice with ethyl acetate. The combined organic layers are then washed successively with water and brine dried over magnesium sulfate, filtered and concentrated to obtain 1-chloroacetyl-2-(S)-cyanopyrrolidine as a yellow solid.

Alternatively, the reaction may be carried out by using, as base, a mixture, e.g. 2-ethyl-hexanoic acid/sodium hydride.

B. Preparation of the title compound in free base form

To a 200 ml flask containing 60 ml of $CH_2Cl_2$ is added 1.85 g (9.27 mmol) of 2-[(5-chloro-2-pyrindinyl)amino]-1,1 -dimethylethylamine and 3.95 g of $K_2CO_3$ and the mixture is cooled in an ice bath. To this cooled mixture is slowly added 1.20g (7.14 mmol) of the above chloride compound prepared in A) dissolved in 30 ml of $CH_2Cl_2$. The resultant mixture is stirred at room temperature for 2 days. The $K_2CO_3$ is then removed via filtration and the filtrate is concentrated via rotovaping. The crude form is then purified on silica gel employing a SIMS/Biotage Flash chromatography system and a 3% solution of methanol in methylene chloride as the eluent to yield the title compound in free base form as a sticky yellow solid.

C. Preparation of the title compound

After dissolving the free base compound prepared in B) above in 20 ml of dry tetrahydrofuran, hydrogen chloride gas is bubbled into the solution for 20 seconds. The reaction was stirred for five minutes and then concentrated via rotovap and then high vacuum pumping to obtain the title compound as an off-white solid, m.p. 164º-166ºC. $^{13}C$ NMR (ppm) = 119.17.

## EXAMPLE 2

Following essentially the procedure of **Example 1**, and using in place of the amine therein an equivalent amount of the above described or commercially available:

l) 1-[4-[(5-cyano-2-pyridinyl)amino]cyclohexyl]amine;
m) 1-[4-[(phenylsulfonyl)amino]cyclohexyl]amine;
n) 1-[4-(benzoylamino)cyclohexyl]amine;
o) 1-[4-[[(4-trifluoromethyl)-2-pyrimidinyl]amino]cyclohexyl]amine;
p) 1-[4-[(3-trifluoromethyl-2-pyridinyl)amino]cyclohexyl]amine;
q) 1-[[4-[(4-chlorophenyl)sulfonyl]amino]cyclohexyl]amine;
r) 1-[4-[(5-trifluoromethyl-2-pyridinyl)amino]cyclohexyl]amine;
s) 1-[4-[(2-chloro-4-pyrimidinyl)amino]cyclohexyl]amine;
t) 1-[4-[(4-chlorobenzoyl)amino]cyclohexyl]amine;
u) 1-[4-[(2,2-dimethyl-1-oxopropyl)amino]oyclohexyl]amine;

v) 1-[4-[(2-benzothiazolyl)amino]cyclohexyl]amine;
w) 1-[4-[(4-cyanophenyl)amino]cyclohexyl]amine;
x) 1-[4-[(cyclohexylcarbonyl)amino]cyclohexyl]amine;
y) 1-[4-[(5-chloro-2-benzothiazolyl)amino]cyclohexyl]amine;
z) 1-[4-[[[(4-trifluoromethyl)phenyl]sulfonyl]amino]cyclohexyl]amine;
aa) 1-[4-[[(2-thienyl)sulfonyl]amino]cyclohexyl]amine;
ee) 1-[4-[4-(trifluoromethyl)phenoxy]cydohexyl]amine;
ff) 1-[[4-[4-(chlorophenoxy)]cyclohexyl]amine;
gg) 1-[4-[(3-trifluoromethyl)phenoxy]cyclohexyl]amine;
hh) 1-[4-(3-chlorophenoxy)cyclohexyl]amine,

there is obtained the following products as hydrochloride salt or, if (C) in **Example 1** is not performed, the free base:

L) 1-[[[4-[(5-cyano-2-pyridinyl)amino]cyclohexyl]amino]acetyl]-2-cyano-(S)-pyrrolidine, dihydrochloride as a white solid (melting point = 242°-244°C, $^{13}C$ NMR $\delta$ 119.31 ppm (CN));
M) 1-[[[4-[(phenylsulfonyl)amino]cyclohexyl]amino]acetyl]-2-cyano-(S)-pyrrolidine, monohydrochloride as a white solid (melting point = 120°-122°C, $^{13}C$ NMR $\delta$ 119.25 ppm (CN));
N) 1-[[[4-(benzoylamino)cyclohexyl]amino]acetyl]-2-cyano-(S)-pyrrolidine, free base as a white fluffy solid (melting point = 78°-80°, $^{13}C$ NMR $\delta$ 119.68 ppm (CN));
O) 1-[[[4-[[(4-trifluoromethyl)-2-pyrimidinyl]amino]cyclohexyl]amino]acetyl]-2-cyano-(S)-pyrrolidine, dihydrochloride as a white solid (decomposed >300°C, $^{13}C$ NMR $\delta$ 119.97 ppm (CN));
P) 1-[[4-[[(3-trifluoromethyl-2-pyridinyl)amino]cyclohexy]amino]acetyl-2-cyano-(S)-pyrrolidine, dihydrochloride as an off-white solid (melting point = 289°-292°C, $^{13}C$ NMR $\delta$ 119.65 ppm (CN));
Q) 1-[[[[4-[(4-chlorophenyl)sulfonyl]amino]cyclohexyl]amino]acetyl]-2-cyano-(S)-pyrrolidine, monohydrochloride as a white solid (melting point = 160°-162°C, $^{13}C$ NMR $\delta$ 119.19 ppm (CN));
R) 1-[[[4-[(5-trifluoromethyl-2-pyridinyl)amino]cyclohexyl]amino]acetyl]-2-cyano-(S)-pyrrolidine, dihydrochloride as a light yellow solid (melting point = 270°-273°C, $^{13}C$ NMR $\delta$ 119.02 ppm (CN));
S) 1-[[[4-[(2-chloro-4-pyrimidinyl)amino]cyclohexyl]amino]acetyl]-2-cyano-(S)-pyrrolidine, dihydrochloride as a white solid (melting point = 290°-293°C, $^{13}C$ NMR $\delta$ 119.28 ppm (CN));
T) 1-[[[4-[(4-chlorobenzoyl)amino]cyclohexyl]amino]acetyl]-2-cyano-, (S)-pyrrolidine, monohydrochloride as a white solid (melting point = 260°-263°C, $^{13}C$ NMR $\delta$ 119.29 ppm (CN));
U) 1-[[[4-[(2,2-dimethyl-1-oxopropyl)amino]cyclohexyl]amino]acetyl]-2-cyano-(S)-pyrrolidine, monohydrochloride as a white solid (melting point = 290°-294°C, $^{13}C$ NMR $\delta$ 119.3 ppm (CN)).
V) 1-[[[4-[(2-benzothiazolyl)amino]cyclohexyl]amino]acetyl]-2-cyano-(S)-pyrrolidine, dihydrochloride as an off-white solid (melting point = 246°-248°C, $^{13}C$ NMR $\delta$ 119.32 ppm (CN));
W) 1-[[[4-[(4-cyanophenyl)amino]cyclohexyl]amino]acetyl]-2-cyano-(S)-pyrrolidine, dihydrochloride as a white solid (melting point = 165°-167°C, $^{13}C$ NMR $\delta$ 119.29 ppm (CN));
X) 1-[[[4-[(cyclohexylcarbonyl)amino]cyclohexyl]amino]acetyl]-2-cyano-(S)-pyrrolidine monohydrochloride as a white solid (melting point = 189°-190°C, $^{13}C$ NMR $\delta$ 119.34 ppm (CN));
Y) 1-[[[4-[(5-chloro-2-benzothiazolyl)amino]cyclohexyl]amino]acetyl]-2-cyano-(S)-pyrrolidine, dihydrochloride as a white fluffy solid (melting point = 290°-294°C, $^{13}C$ NMR $\delta$ 120.32 ppm (CN));
Z) 1-[[[4-[[[(4-trifluoromethyl)phenyl]sulfonyl]amino]cyclohexyl]amino]acetyl]-2-cyano-(S)-pyrrolidine, monohydrochloride as a very light yellow solid (melting point = 135°-137°C, $^{13}C$ NMR $\delta$ 119.17 ppm (CN));
AA) 1-[[14-[(2-thienyl)sulfonyl]amino]cyolohexyl]amino]aoetyl]-2-cyano-(S)-pyrrolidine, monohydrochloride as a white fluffy solid (melting point = 75°-77°C, $^{13}C$ NMR $\delta$ 119.58 ppm (CN));
EE) 1-[[[4-[4-(trifluoromethyl)phenoxy]cyclohexyl]amino]acetyl]-2-cyano-(S)-pyrrolidine, monohydrochloride as an off-white solid (decomposed > 260°C, $^{13}C$ NMR $\delta$ 119.29 ppm (CN));
FF) 1-[[4-[4-chlorophenoxy)cyclohexyl]amino]acetyl]-2-cyano-(S)-pyrrolidine, monohydrochloride as an off-white solid (melting point = 232°-235°C, $^{13}C$ NMR $\delta$ 119-61 ppm (CN));
GG) 1-[[[4-[(3-trifluoromethyl)phenoxy]cyclohexyl]amino]acetyl]-2-cyano-(S)-pyrrolidine, monohydrochloride as a fluffy, very light-yellow solid (melting point = 120°-122°C, $^{13}C$ NMR $\delta$ 119.23 ppm (CN)):
HH) 1-[[[4-[(3-chlorophenoxy)cyclohexyl]amino]acetyl]-2-cyano-(S)-pyrrolidine, monohydrochloride as a fluffy, light-yellow solid (melting point = 72°-74°C, $^{13}C$ NMR $\delta$ 122.02 ppm (CN)).

**EXAMPLE 3**

1-[[[1-[(4-Chlorophenyl)sulfonyl]-4-piperidinyl]amino]acetyl]-2-cyano-(S)-pyrrolidine, monohydrochloride

A. Preparation of the title compound as free base

To a 200 ml flask containing 75 ml of $CH_2Cl_2$ is added 4.0 g (20.0 mmol) of 1-(tert-butoxycarbonylamino) piperidine and 7.4 g (53.3 mmol) of $K_2CO_3$ and the mixture is cooled in an ice bath. To this cooled mixture is slowly added 2.30 g (13.3 mmol) of the above chloride compound prepared in 1A) dissolved in 30 ml of $CH_2Cl_2$. The resultant mixture is stirred at room temperature for 3 days. The $K_2CO_3$ is then removed via filtration and the filtrate is concentrated via rotovaping. The crude form is then purified on silica gel employing a SIMS/Biotage Flash chromatography system and a 3% solution of methanol in methylene chloride as the eluent to yield the intermediate 1-[[(1-[tert-butoxycarbonylamino]-4-piperidinyl]amino]acetyl]-2-cyano-(S)-pyrrolidine in free base form as a golden oil. Deprotection of this t-boc amine with 4.0 M HCl in dioxane at room temperature for 5 hours yielded the dihydrochloride salt of 1-[[[4-piperidinyl] amino]acetyl]-2-cyano-(S)-pyrrolidine as a white solid. To an ice-cold mixture of this amine (300 mg, 0.97 mmol), 30 ml of $CH_2Cl_2$ and 560 mg (4.02 mmol) of $K_2CO_3$ was slowly added 170 mg (0.81 mmol) of 4-chlorobenzenesulfonyl chloride dissolved in 15 ml $CH_2Cl_2$. The resulting mixture was stirred at ice-cold temperature for 2 hours and then at room temperature for 18 hours. Following an EtOAc/water workup, the crude form is then purified on silica gel employing a SIMS/Biotage Flash chromatograhy system and a 3% solution of methanol in methylene chloride as the eluent to yield the title compound in free base form.

B. Preparation of the title compound

After dissolving the free base compound prepared above in 15 ml 4.0 M HCl in dioxane, the reaction was stirred at room temperature for 5 hours and then concentrated via a rotovap and then a high vacuum pump to obtain the title compound as a light green solid, m.p. 252°-255°C. $^{13}C$ NMR (ppm) = 119.25.

**EXAMPLE 4**

Following essentially the procedure of **Example 3**, and using in place of the **4-**chlorobenzenesulfonyl chloride therein, an equivalent amount of:

a) cyclohexanecarbonyl chloride;

there is obtained:

A) 1-[[[1-(cyclohexylcarbonyl)-4-piperidinyl]amino]acetyl]-2-cyano-(S)-pyrrolidine, monohydrochloride as a white solid (melting point > 300°C, $^{13}C$ NMR δ119.61 ppm (CN));

**EXAMPLE 5**

1-[[[4-1(4-Fluorobenzoyl)amino]cyclohexyl]amino]acetyl]-2-cyano-, S)-pyrrolidine, monohydrochloride

Preparation of the title compound in Free Base Form:

To a 100 ml flask containing 30 ml of THF is added 0.325 g (1.38 mmol) of 1-[4-[(4-fluorobanzoyl)amino] cyclohexyl]amine and 0.285 g of $K_2CO_3$ and the mixture is cooled in an ice bath. To this cooled mixture is slowly added 0.120 g (0.69 mmol) of 1-chloroacety)-2-(S)-cyanopyrrolidine in 10 ml of THF. The resultant mixture is stirred at room temperature for 5 days. The potassium salts are then removed via filtration and the filtrate is concentrated via rotovaping. The crude form is then purified on silica gel employing a SIMS/Biotage Flash chromatography system with a 5% solution of methanol in methylene chloride as the eluent to yield the title compound in free base form as white solid.

Preparation of the Title Compound:

After dissolving the free base compound prepared above in 20 ml of dry ethyl acetate, hydrogen chloride gas is bubbled into the solution for 20 seconds. The reaction was stirred for 15 min and then concentrated via rotovap, washed twice with 10 ml of anhydrous diethyl ether and dried under high vacuum pumping to obtain the title compound as white solid, m.p. 212°-214°C., $^{13}C$ NMR 119.29 ppm (CN));

The starting material can be prepared e.g. as follows:

Synthesis of nucleophile: 1-[4-[(4-fluorobenzoyl)amino]cyclohexyl]amine: To an ice-cold solution of trans-1,4-diaminocyclohexane (4.32 g, 37.9 mmol) and $K_2CO_3$ (7.0 g, 50.5 mmol) in 75 ml of $CH_2Cl_2$ is added a solution of benzoyl chloride (1.5 ml, 12.6 mmol) in 25 ml of $CH_2Cl_2$ over 10 minutes. The resulting mixture is then stirred at ice-water temperature for 2 h. The potassium salts are then removed via filtration and the filtrate is concentrated via rotovaping. The residue is then partitioned between $CH_2Cl_2$ and water.
The product is then extracted into the $CH_2Cl_2$ layer, dried over sodium sulfate and concentrated to obtain 1-[4-[(4-fluorobenzoyl)amino]cyclohexyl]amine as a white solid.

Formulation Example

Tablets, each containing 50 mg of active ingredients, e.g., 1-[[[4-(benzoylamino) cyclohexyl]amino]acetyl]-2-cyano-(S)-pyrrolidine in free base form, can be prepared as follows:

| Composition (for 10,000 tablets) | |
|---|---|
| Active ingredient | 500.0 g |
| Lactose | 500.0 g |
| Potato starch | 352.0 g |
| Gelatin | 8.0 g |
| Talc | 60.0 g |
| Magnesium stearate | 10.0 g |
| Silica (highly disperse) | 20.0 g |
| Ethanol | q.s. |

The active ingredient is mixed with the lactose and 292 g of potato starch, and the mixture is moistened using an alcoholic solution of the gelatin and granulated by means of a sieve. After drying, the remainder of the potato starch, the talc, the magnesium stearate and the highly disperse silica are admixed and the mixture is compressed to give tablets of weight 145.0 mg each and active ingredient content 50.0 mg which, if desired, can be provided with breaking notches for finer adjustment of the dose.

## Claims

1. A compound of the formula

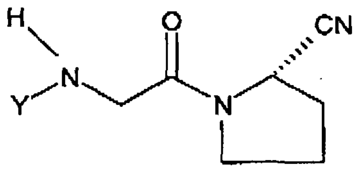

(I)

where Y is selected from the group consisting of:
i) a group of the formula

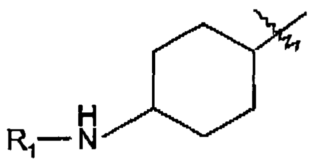

,

where $R_1$
is an unsubstituted pyridine, pyrimidine or phenyl ring; a pyridine, pyrimidine or phenyl ring which is mono- or independently di-substituted by halo, trifluoromethyl, cyano, nitro or $C_{1-6}$alkyl; an unsubstituted phenylsulfonyl group;

a phenylsulfonyl group which is mono- or di-substituted on the phenyl ring by halo, trifluoromethyl, cyano, nitro or $C_{1}$-$_{6}$alkyl; unsubstituted benzoyl; a benzoyl group which is mono- or di-substituted by halo or $C_{1-6}$alkyl; $C_{1-6}$alkylcarbonyl; thienyl sulfonyl; unsubstituted benzothiazole; or a benzothiazole group which is substituted on the phenyl ring by halo or $C_{1-6}$alkyl;

ii) a group of the formula

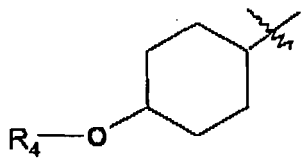

,

where $R_4$ is an unsubstituted phenyl ring; or a phenyl ring which is mono- or di-substituted by halo, trifluoromethyl, cyano, nitro or $C_{1-6}$alkyl; and

iii) a group of the formula

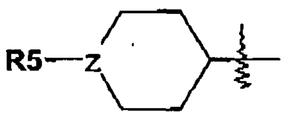

where $R_5$ is $C_{3-8}$cycloalkyl-carbonylamino, and Z is CH; and wherein the bond containing the wavy line signifies the point of attachment of the "Y" group to the glycyl-2-cyanopyrrolidine moiety; or a pharmaceutically acceptable acid addition salt thereof.

**2.** A compound according to claim 1 of the formula

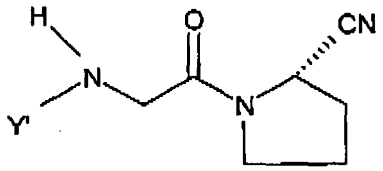

(Ia)

where Y' is selected from the group consisting of:

i) a group of the formula

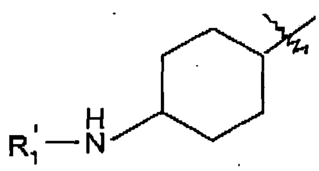

,

where $R_1$' is an unsubstituted pyridine, pyrimidine or phenyl ring; a pyridine, pyrimidine or phenyl ring which is monosubstituted by halo, trifluoromethyl, cyano, nitro or $C_{1-6}$-alkyl; an unsubstituted phenylsulfonyl group; a phenylsulfonyl group which is monosubstituted on the phenyl ring by halo, trifluoromethyl, cyano, nitro or $C_{1-6}$-alkyl; unsubstituted benzoyl; a benzoyl group which is monosubstituted by halo or $C_{1-6}$alkyl; $C_{1-6}$alkylcaronyl; thienyl sulfonyl; unsubstituted benzothiazole; or a benzothiazole group which is substituted on the phenyl ring by halo or $C_{1-6}$alkyl; and

ii) a group of the formula

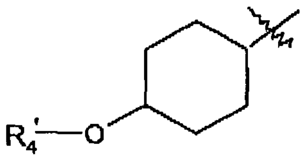

,

where $R_4$'
is an unsubstituted phenyl ring; or a phenyl ring which is monosubstituted by halo, trifluoromethyl, cyano, nitro or $C_{1-8}$alkyl;
or a pharmaceutically acceptable acid addition salt thereof.

**3.** A compound according to claim 2 of the formula

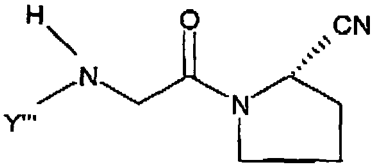

(Ic)

where Y''' is:
i) a group of the formula

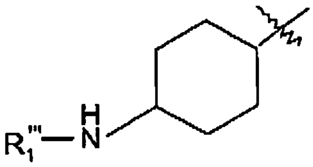

,

where $R_1'''$
is an unsubstituted pyridine, pyrimidine or phenyl ring; a pyridine, pyrimidine or phenyl ring which is monosubstituted by chloro, trifluoromethyl, or cyano; an unsubstituted phenylsulfonyl group; a phenylsulfonyl group which is monosubstituted on the phenyl ring by chloro or trifluoromethyl; unsubstituted benzoyl; a benzoyl group which is monosubstituted by chloro; $C_{1-6}$alkylcarbonyl; thienyl sulfonyl; unsubstituted benzothiazole; or a benzothiazole group which is substituted on the phenyl ring by chloro; and
f) a group of the formula

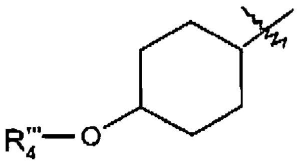

,

where $R_4'''$
is an unsubstituted phenyl ring; or a phenyl ring which is monosubstituted by chloro or trifluoromethyl;
or a pharmaceutically acceptable acid addition salt thereof.

**4.** The compound according to any one of claims 1 to 3, which is selected from the group of the compounds of the formulae

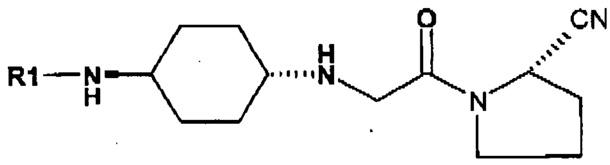

(I-b),

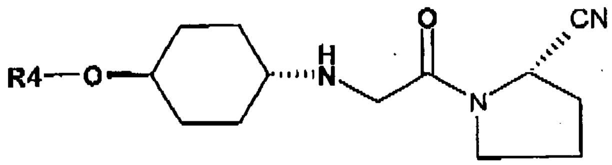

(I-f)

and

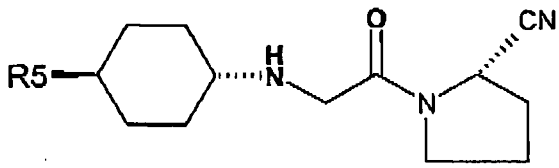

(I-g).

**5.** The compound according to any one of claims 1 to 4, which is selected from the group of compounds consisting of 1-[[[4-(benzoylamino)cyclohexyl]amino]acetyl]-2-cyano-(S)-pyrrolidine and 1-[[[4-[(4-fluorobenzoyl)amino]cyclohexyl]amino]acetyl]-2-cyano-(S)-pyrrolidine, in each case in free base form or in pharmaceutically acceptable acid addition salt form.

**6.** The compound according to claim 5 in free base form.

**7.** A pharmaceutical composition comprising a pharmaceutically acceptable carrier or diluent and a therapeutically effective amount of a compound according to any one of claims 1 to 5 or a pharmaceutically acceptable acid addition salt thereof.

**8.** The use of a compound according to any one of claims 1 to 5 or a pharmaceutically acceptable salt thereof for the manufacture of a medicament for the treatment of non-insulin dependent diabetes mellitus, arthritis, obesity, osteoporosis or further conditions of impaired glucose tolerance.

**9.** A compound according to any one of claims 1 to 5 or a pharmaceutically acceptable acid addition salt thereof for use as a medicament.

**Patentansprüche**

**1.** Verbindungen der Formel

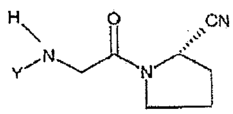

(I),

wobei Y ausgewählt ist aus der Gruppe bestehend aus:

i) einer Gruppe der Formel

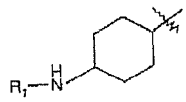

,

wobei $R_1$ für einen unsubstituierten Pyridin-, Pyrimidin- oder Phenylring; einen durch Halogen, Trifluormethyl, Cyano, Nitro oder $C_{1-6}$-Alkyl mono- oder unabhängig disubstituierten Pyridin-, Pyrimidin- oder Phenylring; eine unsubstituierte Phenylsulfonylgruppe; eine am Phenylring durch Halogen, Trifluormethyl, Cyano, Nitro oder $C_{1-6}$-Alkyl mono- oder disubstituierte Phenylsulfonylgruppe; unsubstituiertes Benzoyl; eine durch Halogen oder $C_{1-6}$-Alkyl mono- oder disubstituierte Benzoylgruppe; $C_{1-6}$-Alkylcarbonyl; Thienylsulfonyl; unsubstituiertes Benzothiazol; oder eine am Phenylring durch Halogen oder $C_{1-6}$-Alkyl substituierte Benzothiazolgruppe steht;

ii) einer Gruppe der Formel

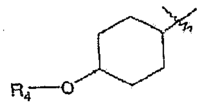

,

wobei $R_4$ für einen unsubstituierten Phenylring ; oder einen durch Halogen, Trifluormethyl, Cyano, Nitro oder $C_{1-6}$-Alkyl mono- oder disubstituierten Phenylring steht; und

iii) einer Gruppe der Formel

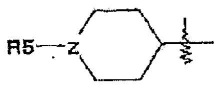

,

wobei $R_5$ für $C_{3-8}$-Cycloalkylcarbonylamino steht und Z für CH steht;
und wobei die die wellenförmige Linie enthaltende Bindung den Anbindungspunkt der "Y"-Gruppe an die Glycyl-2-cyanopyrrolidineinheit bezeichnet;
und deren pharmazeutisch unbedenkliche Säureadditionssalze.

**2.** Verbindungen nach Anspruch 1 der Formel

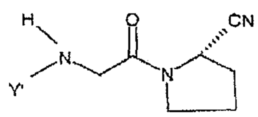

(Ia)

,

wobei Y' ausgewählt ist aus der Gruppe bestehend aus:

i) einer Gruppe der Formel

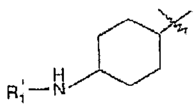

,

wobei $R_1$' für einen unsubstituierten Pyridin-, Pyrimidin- oder Phenylring; einen durch Halogen, Trifluormethyl,

Cyano, Nitro oder $C_{1-6}$-Alkyl monosubstituierten Pyridin-, Pyrimidin- oder Phenylring; eine unsubstituierte Phenylsulfonylgruppe; eine am Phenylring durch Halogen, Trifluormethyl, Cyano, Nitro oder $C_{1-6}$-Alkyl monosubstituierte Phenylsulfonylgruppe; unsubstituiertes Benzoyl; eine durch Halogen oder $C_{1-6}$-Alkyl monosubstituierte Benzoylgruppe; $C_{1-6}$-Alkylcarbonyl; Thienylsulfonyl; unsubstituiertes Benzothiazol; oder eine am Phenylring durch Halogen oder $C_{1-6}$-Alkyl substituierte Benzothiazolgruppe steht; und

ii) einer Gruppe der Formel

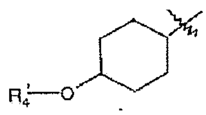

,

wobei $R_4$' für einen unsubstituierten Phenylring; oder einen durch Halogen, Trifluormethyl, Cyano, Nitro oder $C_{1-6}$-Alkyl monosubstituierten Phenylring steht;
und deren pharmazeutisch unbedenkliche Säureadditionssalze.

**3.** Verbindungen nach Anspruch 2 der Formel

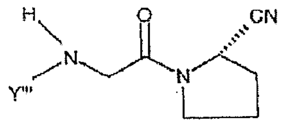

(Ic)

,

wobei Y''' für:

i) eine Gruppe der Formel

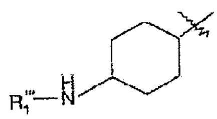

,

wobei $R_1$''' für einen unsubstituierten Pyridin-, Pyrimidin- oder Phenylring; einen durch Chlor, Trifluormethyl oder Cyano monosubstituierten Pyridin-, Pyrimidin- oder Phenylring; eine unsubstituierte Phenylsulfonylgruppe; eine am Phenylring durch Chlor oder Trifluormethyl monosubstituierte Phenylsulfonylgruppe; unsubstituiertes Benzoyl; eine durch Chlor monosubstituierte Benzoylgruppe; $C_{1-6}$-Alkylcarbonyl; Thienylsulfonyl; unsubstituiertes Benzothiazol; oder eine am Phenylring durch Chlor substituierte Benzothiazolgruppe steht; und

f) eine Gruppe der Formel

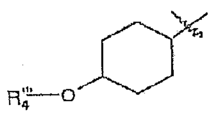

,

wobei $R_4$''' für einen unsnbstituierten Phenylring; oder einen durch Chlor oder Trifluormethyl monosubstituierten Phenylring steht; steht,
und deren pharmazeutisch unbedenkliche Säureadditionssalze.

**4.** Verbindungen nach einem der Ansprüche 1 bis 3, ausgewählt aus der aus den Verbindungen der Formeln

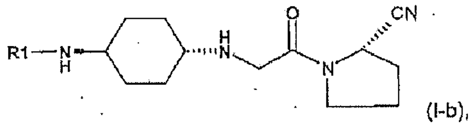

(I-b),

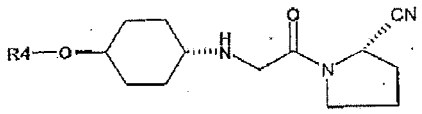

(I-f)

,

und

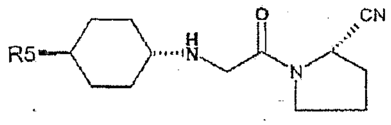

(I-g).

bestehenden Gruppe.

5. Verbindungen nach einem der Ansprüche 1 bis 4, ausgewählt aus der aus den Verbindungen 1-[[[4-(Benzoylamino)cyclohexyl]amino]acetyl]-2-cyano-(*S*) -pyrrolidin und 1-[[[4-[(4-Fluorbenzoyl)amino]cyclohexyl]amino]acetyl]-2-cyano-(S)-pyrrolidin bestehenden Gruppe, jeweils in Form der freien Base oder in Form eines pharmazeutisch unbedenklichen Säureadditionssalzes.

6. Verbindung nach Anspruch 5 in Form der freien Base.

7. Pharmazeutische Zusammensetzung, enthaltend einen pharmazeutisch unbedenklichen Träger oder ein pharmazeutisch unbedenkliches Verdünnungsmittel und eine therapeutisch wirksame Menge einer Verbindung nach einem der Ansprüche 1 bis 5 oder eines pharmazeutisch unbedenklichen Säureadditionssalzes davon.

8. Verwendung einer Verbindung nach einem der Ansprüche 1 bis 5 oder eines pharmazeutisch unbedenklichen Salzes davon zur Herstellung eines Medikaments zur Behandlung von nichtinsulinabhängigem Diabetes mellitus, Arthritis, Obesitas, Osteoporose oder anderen mit gestörter Glukosetoleranz in Zusammenhang stehenden Leiden.

9. Verbindungen nach einem der Ansprüche 1 bis 5 und deren pharmazeutisch unbedenkliche Säureadditionssalze zur Verwendung als Medikament.

## Revendications

1. Composé de formule

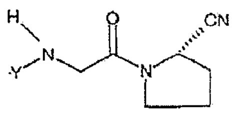

(I)

où Y est choisi dans le groupe constitué par :

i) un groupe de formule

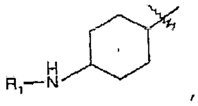

où $R_1$ est un noyau pyridine, pyrimidine ou phényle non substitué ; un noyau pyridine, pyrimidine ou phényle qui est monosubstitué ou disubstitué indépendamment par un halo, trifluorométhyle, cyano, nitro ou alkyle en $C_{1-6}$ ; un groupe phénylsulfonyle non substitué ; un groupe phénylsulfonyle qui est monosubstitué ou disubstitué sur le noyau phényle par un halo, trifluorométhyle, cyano, nitro ou alkyle en $C_{1-6}$ ; un benzoyle non substitué ; un groupe benzoyle qui est monosubstitué ou disubstitué par un halo ou alkyle en $C_{1-6}$ ; un (alkyl en $C_{1-6}$) carbonyle ; un thiénylsulfonyle ; un benzothiazole non substitué ; ou un groupe benzothiazole qui est substitué sur le noyau phényle par un halo ou alkyle en $C_{1-6}$ ;

ii) un groupe de formule

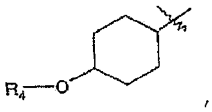

où $R_4$ est un noyau phényle non substitué ; ou un noyau phényle qui est monosubstitué ou disubstitué par un halo, trifluorométhyle, cyano, nitro ou alkyle en $C_{1-6}$ ;

et

iii) un groupe de formule

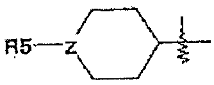

où $R_5$ est un (cycloalkyl en $C_{3-8}$) carbonylamino et Z est CH ; et où la liaison contenant la ligne ondulée représente le point de liaison du groupe « Y » à la fraction glycyl-2-cyanopyrrolidine ;

ou sel d'addition avec un acide pharmaceutiquement acceptable de celui-ci.

**2.** Composé selon la revendication 1 de formule

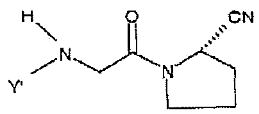

(Ia)

où Y' est choisi dans le groupe constitué par :

i) un groupe de formule

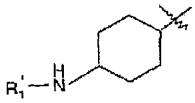

où $R_1$' est un noyau pyridine, pyrimidine ou phényle non substitué ; un noyau pyridine, pyrimidine ou phényle qui est monosubstitué par un halo, trifluorométhyle, cyano, nitro ou alkyle en $C_{1-6}$ ; un groupe phénylsulfonyle non substitué ; un groupe phénylsulfonyle qui est monosubstitué sur le noyau phényle par un halo, trifluorométhyle, cyano, nitro ou alkyle en $C_{1-6}$ ; un benzoyle non substitué ; un groupe benzoyle qui est monosubstitué par un halo ou alkyle en $C_{1-6}$ ; un (alkyl en $C_{1-6}$) carbonyle ; un thiénylsulfonyle ; un benzothiazole non substitué ; ou un groupe benzothiazole qui est substitué sur le noyau phényle par un halo ou alkyle en $C_{1-6}$ ; et

ii) un groupe de formule

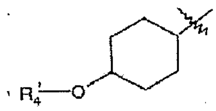

où $R_4$' est un noyau phényle non substitué ; ou un noyau phényle qui est monosubstitué par un halo, trifluorométhyle, cyano, nitro ou alkyle en $C_{1-6}$ ;
ou sel d'addition avec un acide pharmaceutiquement acceptable de celui-ci.

**3.** Composé selon la revendication 2 de formule

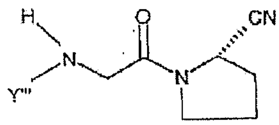

(Ic)

où Y''' est :

1) un groupe de formule

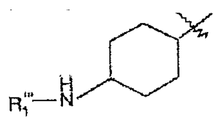

où $R_1$''' est un noyau pyridine, pyrimidine ou phényle non substitué ; un noyau pyridine, pyrimidine ou phényle qui est monosubstitué par un chloro, trifluorométhyle ou cyano ; un groupe phénylsulfonyle non substitué ; un groupe phénylsulfonyle qui est monosubstitué sur le noyau phényle par un chloro ou trifluorométhyle ; un benzoyle non substitué ; un groupe benzoyle qui est monosubstitué par un chloro ; un (alkyl en $C_{1-6}$) carbonyle ; un thiénylsulfonyle ; un benzothiazole non substitué ; ou un groupe benzothiazole qui est substitué sur le noyau phényle par un chloro ; et
f) un groupe de formule

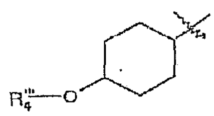

où $R_4$''' est un noyau phényle non substitué ; ou un noyau phényle qui est monosubstitué par un chloro ou trifluorométhyle ;
ou sel d'addition avec un acide pharmaceutiquement acceptable de celui-ci.

**4.** Composé selon l'une quelconque des revendications 1 à 3, qui est choisi dans le groupe des composés répondant aux formules

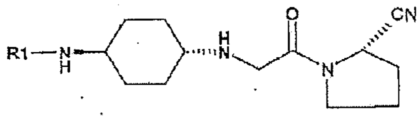

(I-b),

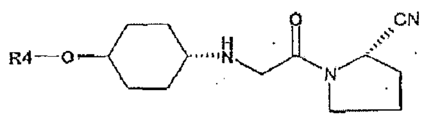

(I-f)

et

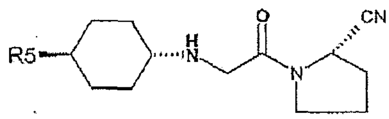

(I-g).

**5.** Composé selon l'une quelconque des revendications 1 à 4, qui est choisi dans le groupe de composés constitué par la 1-[[[4-(benzoylamino)cyclohexyl]amino]açétyl]-2-cyano-(S)-pyrrolidine et la 1-[[[4-[(4-fluorobenzoyl)amino]cyclohexyl]amino]acétyl]-2-cyano-(S)-pyrrolidine, dans chaque cas sous forme de base libre ou sous forme d'un sel d'addition avec un acide pharmaceutiquement acceptable.

**6.** Composé selon la revendication 5 sous forme de base libre.

**7.** Composition pharmaceutique comprenant un vecteur ou diluant pharmaceutiquement acceptable et une quantité thérapeutiquement efficace d'un composé selon l'une quelconque des revendications 1. à 5 ou d'un sel d'addition avec un acide pharmaceutiquement acceptable de celui-ci.

**8.** Utilisation d'un composé selon l'une quelconque des revendications 1 à 5 ou d'un sel pharmaceutiquement acceptable de celui-ci pour la fabrication d'un médicament pour le traitement du diabète sucré non insulinodépendant, de l'arthrite, de l'obésité, de l'ostéoporose ou d'autres affections d'intolérance au glucose.

**9.** Composé selon l'une quelconque des revendications 1 à 5 ou sel d'addition avec un acide pharmaceutiquement acceptable de celui-ci destiné à être utilisé comme médicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9819998 A
- US 6011155 A
- US 6057316 A
- US 6001867 A
- WO 9958518 A
- WO 9958522 A
- WO 9946268 A
- WO 9946267 A
- WO 9946244 A
- WO 9946237 A
- WO 99146236 A
- WO 99115529 A
- WO 9958127 A
- WO 0014090 A
- WO 9940062 A
- WO 9840385 A
- EP 682024 A
- WO 0014095 A
- WO 9947549 A
- WO 9839344 A
- WO 9839343 A
- WO 9839342 A
- EP 978279 A
- US 5998463 A
- WO 9926659 A
- EP 846464 A
- WO 97131901 A
- WO 9639384 A
- WO 9639385 A
- WO 9804528 A
- US 5880139 A
- WO 99101423 A
- US 5776954 A
- WO 9822109 A
- WO 9822108 A
- WO 9821957 A
- WO 9716442 A
- US 6030837 A
- WO 0021927 A
- WO 9741854 A
- US 4981784 A
- US 5071773 A
- US 5298429 A
- US 5506102 A
- WO 8905355 A
- WO 9106677 A
- WO 9205447 A
- WO 93111235 A
- WO 9518380 A
- US 9304399 W
- US 9403795 W
- CA 2034220
- WO 9605165 A
- US 9516842 W
- US 9516695 W
- US 9310094 W
- WO 9415901 A
- US 9211214 W
- WO 9311755 A
- US 9310166 W
- US 9310204 W
- WO 9415902 A
- US 9303944 W
- WO 9321146 A
- US 60004897 B
- US 60009884 B
- WO 9929672 A
- WO 9832753 A
- WO 9820005 A
- WO 9809625 A
- WO 9746556 A
- WO 9737646 A
- US 5705515 A
- EP 0193256 A1
- EP 0306228 A1
- EP 0139421 A
- EP 0207605 B1
- JP 10087641 A
- EP 0604983 B1
- EP 0332332 A
- US 4997948 A
- US 4287200 A
- EP 0332332 B1
- WO 9908501 A
- US 5866563 A
- US 5705483 A
- US 5120712 A
- US 5118666 A
- US 5512549 A
- WO 9111457 A
- WO 0078726 A
- EP 196222 A
- EP 526171 A
- EP 0147850 A2
- EP 0207331 A1
- EP 0526171 B1
- US 5488510 A
- US 4062950 A

- EP 0226121 A

**Non-patent literature cited in the description**


- **Reisher et al.** Increased expression of intestinal cell line Caco-2. *Proc. Natl. Acad. Sci.,* 1993, vol. 90, 5757-5761
- Involvement of dipeptidylpeptidase IV in an in vivo immune response. *Clin. Exp. Immunol.,* 1992, vol. 89, 192-197
- **Poucher et al.** *Mol. Cell Biochem.,* 1998, vol. 188, 73-80
- *Science,* 1999, vol. 284, 974-97L-783, 281
- *Mol. Cell. Endocrinol.,* 1997, vol. 135 (1), 67-77
- *Diabetes,* 1998, vol. 47, 1630-1636
- *Proc. Natl. Acad Sci USA,* 1998, vol. 95, 1776-1781
- *Bioorg Med. Chem. Lett,* 1992, vol. 2, 915-918CP-99, 711
- *J. Med. Chem.,* 1998, vol. 41, 5150-5157NNC 92-1687
- *J. Biol Chem.,* 1999, vol. 274, 8694-8697L-168, 049
- *Mol. Biol. Diabetes,* 1994, vol. 2, 283-99
- **Aicher et al.** *J. Med. Chem.,* 1999, vol. 42, 2741-2746
- **Boehm et al.** *J. Med. Chem.,* 1994, vol. 38 (16), 3146-3155
- **Boehm et al.** *J. Med. Chem.,* 1994, vol. 37 (18), 2930-2941
- **Antras et al.** *J. Biol. Chem.,* 1991, vol. 266, 1157-1161
- **Salazar-Olivo et al.** *Biochem. Biophys. Res. Commun.,* vol. 204, 157-263
- **Safanova.** *Mol. Cell. Endocrin.,* 1994, vol. 104, 201-211
- **Vidal-Puig et al.** *Biochem. Biophys. Res. Commun.,* 1997, vol. 235 (1), 79-82
- **B.B. Lohray et al.** *J. Med. Chem.,* 1998, vol. 41, 1619-1630
- **J. Wrobel et al.** *J. Med. Chem.,* 1998, vol. 41, 1084-1091
- **Fukui.** *Diabetes,* 2000, vol. 49 (5), 759-767
- **Emil A. Wemer ; James Bell.** *J. Chem. Soc,* 1922, vol. 121, 1790-1794
- **T. Page et al.** *Br. J. Pharmacol.,* 1997, vol. 122, 1464-1468
- **W.E. Schmidt et al.** *Diabetologia,* 1985, vol. 28, 704-707
- **C. Orskov et al.** *J. Biol. Chem.,* 1989, vol. 264, 12826
- **Greig et al.** *Diabetologia,* 1999, vol. 42, 45-50
- **Wang et al.** *J. Pharmacol. Exp. Ther.,* 1996, vol. 278, 82-89
- *J. Clin. Endocrinol. Metab.,* 2000, vol. 85 (3), 1043-1048CCK-8
- *Diabetes Care,* 1998, vol. 21, 897-893
- **O.G. Kolterman et al.** *Diabetologia,* 1996, vol. 39, 492-499
- *Diabetes,* 1987, vol. 36, 216-220